(19) Europäisches Patentamt
European Patent Office
Office européen des brevets

(11) Veröffentlichungsnummer : **0 262 096 B1**

(12) **EUROPÄISCHE PATENTSCHRIFT**

(45) Veröffentlichungstag der Patentschrift : **02.01.92 Patentblatt 92/01**

(21) Anmeldenummer : **87810545.1**

(22) Anmeldetag : **21.09.87**

(51) Int. Cl.$^5$ : **C07D 409/12,** C07D 403/12, C07D 405/12, C07D 253/06, C07D 239/47, A01N 47/36

(54) **Aminopyrazinone und Aminotriazinone.**

(30) Priorität : **26.09.86 CH 3871/86**

(43) Veröffentlichungstag der Anmeldung : **30.03.88 Patentblatt 88/13**

(45) Bekanntmachung des Hinweises auf die Patenterteilung : **02.01.92 Patentblatt 92/01**

(84) Benannte Vertragsstaaten : **AT BE CH DE ES FR GB GR IT LI NL SE**

(56) Entgegenhaltungen :
**EP-A- 0 165 753**
**EP-A- 0 177 163**
**EP-A- 0 187 470**
**CHEMICAL ABSTRACTS, Band 98, Nr. 7, 14. Februar 1983, Columbus, Ohio, USA; LEE, TZOONG CHYH; CHELLO, PAUL L.; CHOU, TING CHAO; TEMPLETON, MARY AGNES; PARHAM, JAMES C. "Synthesis of an N-aminopyrazinonium analog of cytidine." Seite 720, Spalte 2, Zusammenfassung-Nr. 54 382t**
**CHEMICAL ABSTRACTS, Band 103, Nr. 15, 14. Oktober 1985, Columbus, Ohio, USA; BHATTACHARYA, B.K.; EIRICH, F.R. "Synthesis and mass spectral studies of 1-[5-chloro-1-substituted-2(1H)-pyrazin-2-on-3-yl]-5-aryl-3-methylpyrazoles." Seite 724, Spalte 2, Zusammenfassung-Nr. 123 436z**
**CHEMICAL ABSTRACTS, Band 71, Nr. 21, 24. November 1969, Columbus, Ohio, USA; BERNARDI, LUIGI; LUINI ; F.; PALAMIDESSI, G. "Pyrazine derivatives. XII. Sulfanilamidodimethoxypyrazines." Seite 348, Spalte 1, Zusammenfassung-Nr. 101 816s**
**CHEMICAL ABSTRACTS, Band 100, Nr. 21, 21. Mai 1984, Columbus, Ohio, USA; NEUNHOEFFER, HANS; HAMMANN, HEINZ; "Synthesis of 1,2,4-triazines. XI. Synthesis and reactions of 6-amino-1,2,4-triazin-5-ones and 6-amino-1,2,4-triazine-5-thiones." Seite 625, Spalte 1, Zusammenfassung-Nr. 174 740g**

(56) Entgegenhaltungen :
**CHEMICAL ABSTRACTS, Band 99, Nr. 19, 7. November 1983, Columbus, Ohio, USA; VEKEMANS, J.; POLLERS-WIEERS, C.; HOORNAERT, G. "Anew synthesis of substituted 2(1H)-pyrazinones." Seite 615, Spalte 1, Zusammenfassung-Nr. 158 384g**

(73) Patentinhaber : **CIBA-GEIGY AG**
**Klybeckstrasse 141**
**CH-4002 Basel (CH)**

(72) Erfinder : **Böhner, Beat, Dr.**
**Hügelweg 3**
**CH-4102 Binningen (CH)**
Erfinder : **Meyer, Willy**
**Talweg 49**
**CH-4125 Riehen (CH)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

## Beschreibung

Die vorliegende Erfindung betrifft neue, herbizid wirksame und pflanzenwuchsregulierende Aminopyrazinone und Aminotriazinone, Verfahren zu ihrer Herstellung, die sie als Wirkstoffe enthaltende Mittel sowie deren Verwendung zum Bekämpfen von Unkräutern, vor allem selektiv in Nutzpflanzenkulturen oder zum Regulieren und Hemmen des Pflanzenwachstums. Ferner betrifft die Erfindung auch innerhalb des Erfindungskonzeptes hergestellte Zwischenprodukte.

Die erfindungsgemässen Aminopyrazinone und Aminotriazinone entsprechen der Formel I

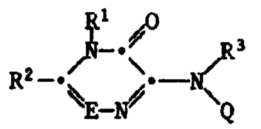

(I),

worin

E Sticksoff oder $=CR^4-$,

$R^1$ $C_1$-$C_4$-Alkyl,

$R^2$ Wasserstoff, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, $C_1$-$C_3$-Alkylsulfinyl, $C_1$-$C_2$-Alkoxyäthoxy, $C_1$-$C_3$-Alkylsulfonyl, Halogen oder $-NR^5R^6$,

$R^3$ Wasserstoff oder $C_1$-$C_3$-Alkyl und

Q eine Gruppe

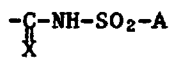

bedeuten,

wobei

$R^4$ für Wasserstoff, $C_1$-$C_3$-Alkyl, $C_1$-$C_3$-Halogenalkyl, $C_1$-$C_3$-Alkoxy, Cyclopropyl, $C_1$-$C_3$-Halogenalkoxy, $C_1$-$C_3$-Alkylthio, $C_2$-$C_4$-Alkoxyalkyl, $C_3$-$C_5$-Dialkoxymethyl, Halogen oder $-NR^5R^6$,

$R^5$ und $R^6$ unabhängig voneinander für Wasserstoff oder $C_1$-$C_3$-Alkyl,

X für Sauerstoff oder Schwefel und

A für eine Gruppe

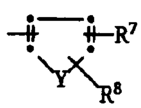

stehen, worin

Y Sauerstoff, Schwefel, $-CH=CH-$, $-NR^9-$ oder $-CR^{10}=N-$,

$R^7$ Wasserstoff, Halogen, $C_1$-$C_3$-Alkyl, $C_1$-$C_3$-Alkoxy, Nitro oder Trifluormethyl,

$R^8$ Wasserstoff, Halogen, $C_1$-$C_3$-Alkyl, $C_1$-$C_3$-Alkoxy, Nitro, $-C\equiv CH$, oder eine der Gruppen

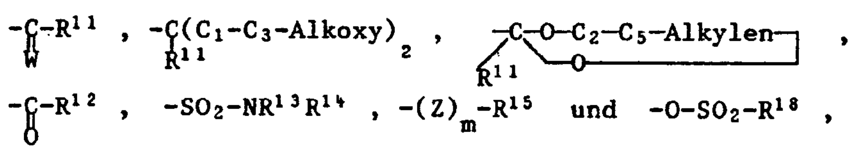

$R^9$ und $R^{10}$ unabhängig voneinander Wasserstoff, $C_1$-$C_3$-Alkyl oder $C_2$-$C_4$-Alkenyl,

$R^{11}$ Wasserstoff, $C_1$-$C_3$-Alkyl, $C_1$-$C_3$-Halogenalkyl, $C_3$-$C_6$-Cycloalkyl oder $C_2$-$C_4$-Alkoxyalkyl,

$R^{12}$ $C_1$-$C_6$-Alkoxy, $C_3$-$C_6$-Alkenyloxy, $C_3$-$C_6$-Alkinyloxy, $C_2$-$C_6$-Halogenalkoxy, $C_1$-$C_4$-Cyanoalkoxy, $C_1$-$C_6$-Alkylthio, $C_3$-$C_6$-Alkenylthio, $C_3$-$C_6$-Alkinylthio, $C_5$-$C_6$-Cycloalkoxy, $C_2$-$C_6$-Alkoxyalkoxy oder $-NR^{16}R^{17}$,

$R^{13}$ Wasserstoff, $C_1$-$C_3$-Alkyl oder $C_3$-$C_4$-Alkenyl,

$R^{14}$ Wasserstoff, $C_1$-$C_3$-Alkyl, $C_1$-$C_3$-Cyanoalkyl oder $C_1$-$C_3$-Alkoxy,

$R^{15}$ $C_3$-$C_6$-Alkinyl, $C_2$-$C_6$-Alkenyl, $C_1$-$C_6$-Alkyl, $C_1$-$C_4$-Halogenalkyl, $C_2$-$C_4$-Halogenalkenyl, $C_1$-$C_4$-Alkyl sub-

stituiert durch Cyano, Methoxy, Aethoxy, Nitro, $C_1$-$C_4$-Alkoxycarbonyl, Methylthio, Aethylthio, Methylsulfonyl oder Aethylsulfonyl; oder $C_3$-$C_4$-Alkenyl substituiert durch Nitro, Cyano, Methoxy oder Aethoxy,

$R^{16}$ Wasserstoff, $C_1$-$C_3$-Alkyl oder $C_3$-$C_4$-Alkenyl,

$R^{17}$ Wasserstoff, $C_1$-$C_3$-Alkyl, $C_1$-$C_3$-Cyanoalkyl oder $C_1$-$C_3$-Alkoxy,

$R^{18}$ $C_1$-$C_3$-Alkyl und $C_1$-$C_3$-Halogenalkyl,

W Sauerstoff oder Schwefel,

Z Sauerstoff, Schwefel, -SO- oder $-SO_2-$, und

m die Zahl null oder eins bedeuten,

sowie den Salzen dieser Verbindungen.

Triazinverbindungen und Pyrimidinverbindungen mit herbizider Wirkung sind allgemein bekannt. Kürzlich wurden derartige Verbindungen mit herbizider und pflanzenwuchsregulierender Wirkung, beispielsweise in den veröffentlichten europäischen Patentanmeldungen 44 807, 44 808 und 126 711 beschrieben.

In den Definitionen ist unter Alkyl geradkettiges oder verzweigtes Alkyl zu verstehen; z.B.: Methyl, Aethyl, n-Propyl, i-Propyl oder die vier isomeren Butyl.

Unter Alkoxy ist zu verstehen: Hethoxy, Aethoxy, n-Propyloxy, i-Propyloxy oder die vier isomeren Butyloxy, insbesondere aber Methoxy, Aethoxy oder i-Propyloxy.

Beispiele für Alkylthio sind Methylthio, Aethylthio, n-Propylthio, i-Propylthio oder die vier isomeren Butylthio, insbesondere aber Methylthio und Aethylthio.

Unter Halogen selbst sowie als Teil eines Substituenten wie in Halogenalkoxy, Halogenalkylthio oder Halogenalkyl sind Fluor, Chlor und Brom, vorzugsweise jedoch Fluor und Chlor zu verstehen. Halogenalkyl selbst oder als Teil von Halogenalkoxy oder Halogenalkylthio steht in der Regel für Chlormethyl, Fluormethyl, Difluormethyl, Tri- fluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3, 3, 3-Hexafluorpropyl, insbesondere aber Fluormethyl, Chlormethyl, Difluormethyl und Trifluormethyl.

Beispiele für Alkoxyalkyl sind: Methoxymethyl, Methoxyäthyl, Methoxypropyl, Aethoxyäthyl, Aethoxymethyl oder Propyloxymethyl. Beispiele für Alkoxyalkoxy sind: Methoxymethoxy, Methoxyäthoxy, Methoxypropyloxy, Aethoxymethoxy, Aethoxyäthoxy sowie Propyloxymethoxy. Alkylengruppen sind im Rahmen der Definition unter Formel I Aethylen, Propylen, Butylen, 1-Methyläthylen, 1-Aethyläthylen, 2-Methylbutylen oder 1-Methylbutylen. Dialkoxymethyl steht vorzugsweise für Dimethoxymethyl oder Diäthoxymethyl.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeigneter Amine sind primäre, sekundäre und tertiäre aliphatische und aromatische Amine wie Methylamin, Aethylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Träthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin, Diäthanolamin und 1,4-Diazabicyclo[2.2.2]octan.

Beispiele für quaternäre Ammoniumbasen sind im allgemeinen die Kationen von Halogenammoniumsalzen, z.B. das Tetramethylammoniumkation, das Tetraäthylammoniumkation, das Trimethyläthylammoniumkation, aber auch das Ammoniumkation.

Unter den Verbindungen der Formel I sind diejenigen bevorzugt, in denen entweder

a) X Sauerstoff bedeutet, oder

b) A für die Gruppe

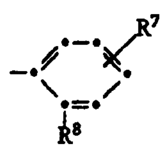

steht, oder

c) $R^3$ Wasserstoff bedeutet, oder

d) $R^1$ Methyl oder Aethyl, $R^2$ Wasserstoff, Methoxy oder Methyl und E die Gruppe $=CR^4$-bedeuten, oder

e) $R^1$ Methyl oder Aethyl, $R^2$ Methoxy, Aethoxy, Methylthio, Dimethylamino, Methyl, Trifluormethyl, 2,2,2-Trifluoräthoxy oder Aethyl und E Stickstoff bedeuten.

Unter den Verbindungen der Gruppe b) sind solche bevorzugt, worin $R^7$ Wasserstoff und $R^8$ Methoxycar-

bonyl, Aethoxycarbonyl, Dimethylaminosulfonyl, Aethoxy, Propoxy, Difluormethoxy, Trifluormethyl, Chloräthoxy, Methoxyäthoxy, 2,2,2-Trifluoräthoxy, 1,2-Dichlorvinyloxy, Nitro, Fluor, Chlor, Brom, Methyl, Methylthio, Difluormethylthio, Chloräthylthio, -O-$SO_2CH_3$, Allyloxy oder Methoxy bedeuten.

Unter den Verbindungen der Gruppe d) sind diejenigen bevorzugt, in denen $R^4$ Chlor, Brom, Methoxy, Aethoxy, Methyl, Aethyl, Methylthio, Dimethylamino, Trifluormethyl oder 2,2,2-Trifluoräthoxy bedeutet.

Besonders bevorzugte Untergruppen von Verbindungen der Formel I werden von denjenigen Verbindungen gebildet, in denen entweder $R^1$ Methyl oder Aethyl, $R^2$ Wasserstoff, Methoxy oder Methyl,

$R^3$ Wasserstoff, X Sauerstoff, A die Gruppe

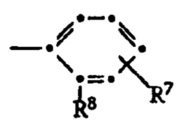
,

$R^7$ Wasserstoff, $R^8$ Methoxycarbonyl, Aethoxycarbonyl, Dimethylaminosulfonyl, Methoxy, Aethoxy, Difluormethoxy, Trifluormethyl, Chloräthoxy, Methoxyäthoxy, 2,2,2-Trifluoräthoxy, 1,2-Dichlorvinyloxy, Nitro, Fluor, Chlor, Brom, Methyl, Methylthio, Difluomethylthio, Chloräthylthio, -O-$SO_2CH_3$ oder Allyloxy, E die Gruppe =$CR^4$- und $R^4$ Chlor, Brom, Methoxy, Aethoxy, Methyl, Aethyl, Methylthio, Dimethylamino, Trifluormethyl oder 2,2,2-Trifluoräthoxy bedeuten; oder in denen

$R^1$ Methyl oder Aethyl, $R^2$ Methoxy, Aethoxy, Methylthio, Dimethylamino, Methyl, Trifluomethyl, 2,2,2-Trifluoräthoxy oder Aethyl,

$R^3$ Wasserstoff, E Stickstoff, X Sauerstoff, A die Gruppe

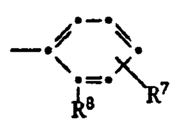
,

$R^7$ Wasserstoff und $R^8$ Methoxycarbonyl, Aethoxycarbonyl, Dimethylaminosulfonyl, Methoxy, Aethoxy, Difluormethoxy, Trifluormethyl, Chloräthoxy, Methoxyäthoxy, 2,2,2-Trifluoräthoxy, 1,2-Dichlorvinyloxy, Nitro, Fluor, Chlor, Brom, Methyl, Methylthio, Difluormethylthio, Chloräthylthio, -O-$SO_2CH_3$ oder Allyloxy bedeuten.

Als bevorzugte Einzelverbindungen der Formel I sind zu nennen:

6-[3-(2-Methoxycarbonylphenylsulfonyl)-ureido]-5-chlor-1-methyl-pyrazin-2-on und

6-[3-(2-Methoxycarbonylphenylsulfonyl)-ureido]-3-methoxy-4-methyl-1,2,4-triazin-5-on.

Die Herstellung der Verbindungen der Formel I erfolgt in allgemeinen nach den folgenden Methoden.

Nach einem ersten Verfahren werden die Verbindungen der Formel I erhalten, indem man ein Aminopyrazinon oder Aminotriazinon der Formel II

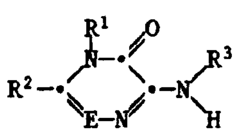
(II)

worin $R^1$, $R^2$, $R^3$ und E die unter Formel I angegebene Bedeutung haben mit einem Sulfonylisocyanat der Formel III

$$X = C = N\text{-}SO_2\text{-}A \quad (III)$$

worin A und X die unter Formel I gegebenen Bedeutungen haben, umsetzt.

Nach einem zweiten Verfahren gelangt man zu den Verbindungen der Formel I, indem man ein Aminopyrazinon oder Aminotriazinon der Formel II in Gegenwart einer Base mit einem Sulfonylcarbamat der Formel IV

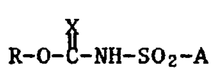
(IV)

worin A und X die unter Formel I gegebene Bedeutung haben und R für Phenyl, Alkyl oder substituiertes Phenyl steht, umsetzt.

Schliesslich kann man die Verbindungen der Formel I auch erhalten, indem man ein Carbamat der Formel

V

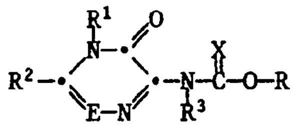

(V)

worin $R^1$, $R^2$, $R^3$, E und X die unter Formel I angegebene Bedeutung haben und R für Phenyl, Alkyl oder substituiertes Phenyl steht, in Gegenwart einer Base mit einem Sulfonamid der Formel VI

$H_2N\text{-}SO_2\text{-}A$ (VI)

worin A die unter Formel I gegebene Bedeutung hat, umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze überführt werden. Dieses geschieht beispielsweise durch Umsetzen mit der äquimolaren Menge Base und Verdampfen des Lösungsmittels.

Die Umsetzungen zu Verbindungen der formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, Tetrachlorkohlenstoff, oder Chorbenzol, Aether wie Diäthyläther, Aethylenglykoldimethyläther, Diäthylenglykoldimethyläther, Tetrahydrofuran oder Dioxan, Nitrile wie Acetonitril oder Propionitril, Amide wie Direthylformamid, Diäthylformamid oder N-Methylpyrrolidinon. Die Reaktionstemperaturen liegen vorzugsweise zwischen -20° und +120°C. Die Umsetzungen der Kupplungsverfahren verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triäthylamin, Chinuclidin, 1,4-Diazabicyclo[2.2.2]-octan, 1,5-Diazabicyclo-[4.3.0]non-5-en oder 1,8-Diazabicyclo[5.4.0]undec-7-en geeignet. Als Basen können aber auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium-und Natriumhydrogencarbonat verwendet werden.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Aether, aromatischen Kohlenwasserstoffen oder chlorierten Kohlenwasserstoffen gereinigt werden.

Die Zwischenprodukte der Formeln III, IV und VI und ihre Herstellung sind aus der Literatur bekannt. Beispielsweise sind diese Substanzen im US-PS 4 127 405, US- 4 305 884, EP-A- 13 480, EP-A- 44 807 und EP-A-44 808 beschrieben.

Die als Ausgangsmaterialien verwendeten Aminopyrazinone und Aminotriazinone der Formel II sind teilweise aus der Literatur bekannt. Die neuen Verbindungen der Formel II wurden speziell für die Herstellung der erfindungsgemässen Wirkstoffe entwickelt und hergestellt. Sie bilden daher einen Teil der vorliegenden Erfindung. Diese Verbindungen entsprechen der Formel II, mit der Massgabe, dass $R^3$ $C_1$-$C_3$-Alkyl bedeutet, wenn

a) gleichzeitig E Stickstoff, $R^1$ Methyl, $R^2$ Methyl oder Methylthio; oder

b) gleichzeitig $R^1$ Methyl, $R^2$ Wasserstoff und E die Gruppe =CCl- oder =CH- bedeuten.

Die Carbamate der Formel V werden aus den Aminoverbindungen der Formel II durch Umsetzung mit Kohlensäure-Derivaten der Formel VII

$(RO)_2C = X$ (VII)

worin R und X die angegebenen Bedeutungen haben, erhalten.

Die Kohlensäure-Derivate der Formel VII sind bekannt und zum Teil im Handel erhältlich.

Die Ausgangsmaterialien der Formel II können je nach angestrebter Substitution nach unterschiedlichen Methoden hergestellt werden.

So erhält man die Aminotriazinone der Unterformel IIa

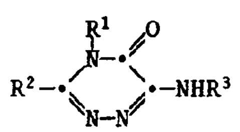

(IIa)

worin $R^1$ und $R^3$ die unter Formel I gegebene Bedeutung haben und $R^2$ $C_1$-$C_3$-Alkyl, $C_1$-$C_3$-Halogenalkyl,

Wasserstoff oder $C_1$-$C_3$-Alkylthio bedeuten, indem man eine Verbindung der Formel VIII

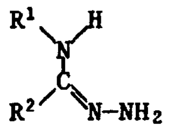

(VIII)

worin $R^1$ und $R^2$ die unter Formel IIa gegebenen Bedeutungen haben, mit einem Oxalsäurederivat der Formel IX

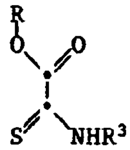

(IX)

worin $R^3$ die unter Formel II gegebene Bedeutung hat und R für Alkyl oder Aryl steht, umsetzt.

Aminotriazinonsynthesen dieses Typs sind in Liebigs Ann. Chem. 1984, 283-295 beschrieben. Die Zwischenprodukte VIII und IX sind bekannt oder können analog zu publizierten Verfahren erhalten werden.

Die Aminotriazinone der Unterformel IIa, worin $R^1$ und $R^3$ die unter Formel I gegebene Bedeutung haben und $R^2$ $C_1$-$C_3$-Alkoxy, $C_2$-$C_3$-Halogenalkoxy, Halogen und $-NR^5R^6$ bedeuten, erhält man, indem man Aminotriazinone der Formel IIa worin $R^2$ $C_1$-$C_3$-Alkylthio bedeutet, mit geeigneten Oxidationsmitteln wie $H_2O_2$, $Cl_2$ oxydiert und anschliessend mit Nucleophilen der Formel X

H-R (X)

worin R $C_1$-$C_3$-Alkoxy, $C_2$-$C_3$-Halogenalkoxy oder $-NR^5R^6$ bedeuten, gegebenenfalls in Gegenwart einer Base umsetzt.

Die Aminopyrazinone der Unterformel IIb

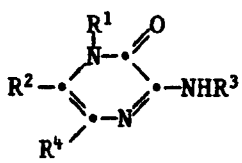

(IIb)

worin $R^1$, $R^2$, $R^3$ und $R^4$ die unter Formel I gegebene Bedeutung haben, werden hergestellt, indem man ein 2-Amino-pyrazinon der Formel XI

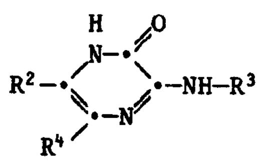

(XI)

worin $R^2$, $R^3$ und $R^4$ die unter Formel I gegebene Bedeutung haben, mit Alkylierungsmitteln wie z.B. Alkylhalogeniden, Alkyltosylaten oder Dialkylsulfaten in Gegenwart einer Base umsetzt. Alkylierungen dieses Typs sind beispielsweise in J. Chem. Soc. 1965, 6681 beschrieben. Ausgangsprodukte der Formel XI sind bekannt oder können analog zu publizierten Methoden erhalten werden.

Aminopyrazine der Formel IIb erhält man auch, indem man Pyrazinone der Formel XII

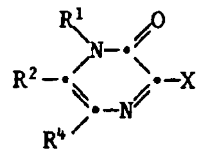

(XII)

worin $R^1$, $R^2$ und $R^4$ die unter Formel I gegebene Bedeutung haben und X für Cl, Br oder $OSO_2CF_3$ steht, mit einem Amin der Formel XIII

$H_2N-R^3$ (XIII)

worin $R^3$ Wasserstoff oder $C_1$-$C_3$-Alkyl bedeuten, umsetzt. Synthesen dieses Typs sind in J. Het. Chem. 20, 919-923 (1983) beschrieben.

Pyrazinone der Unterformel XIIa

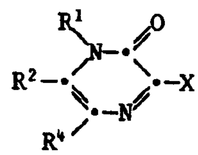

(XIIa)

worin $R^2$ Wasserstoff oder $C_1$-$C_4$-Alkyl, $R^4$ $-NR^5R^6$, $C_1$-$C_3$-Alkylthio, $C_1$-$C_3$-Alkoxy, $C_2$-$C_3$-Halogenalkoxy und $R^1$ und X die in Formel XII gegebene Bedeutung haben, werden hergestellt, indem man Pyrazinone der Formel XIV

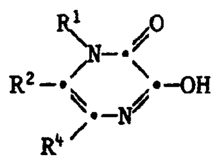

(XIV)

worin $R^1$, $R^2$ und $R^4$ die in Formel XIIa gegebene Bedeutung haben, gegebenenfalls in Gegenwart einer Base in inerten Lösungsmitteln wie zum Beispiel Dichlormethan, Acetonitril, Chloroform oder Tetrachlorkohlenstoff mit $(CF_3SO_2)O$, $PCl_5$, $POCl_3$, $POBr_3$, $(COCl)_2$ oder $(COBr)_2$ umsetzt.

Pyrazinone der Formel XIV, beziehungsweise deren Alkalisalze,erhält man durch Acylierung von Aminoalkylcyaniden der Formel XV

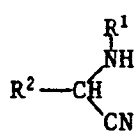

(XV)

worin $R^1$ $C_1$-$C_4$-Alkyl und $R^2$ Wasserstoff oder $C_1$-$C_3$-Alkyl bedeuten, mit Oxalsäurechloriden der Formel XVI

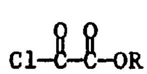

(XVI)

worin R $C_1$-$C_3$-Alkyl bedeutet, und anschliessenden Umsatz mit Nukleophilen der Formel XVII,

$H-R^4$ (XVII)

worin $R^4$ die gleiche Bedeutung hat wie in Formel XIIa, gegebenenfalls in Gegenwart einer Base.

Die Pyrazinone der Unterformel XIIb

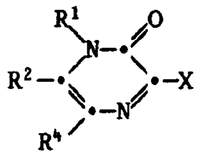

(XIIb)

worin $R^1$ $C_1$-$C_4$-Alkyl, $R^2$ Wasserstoff oder $C_1$-$C_3$-Alkyl, X und $R^4$ Chlor oder Brom bedeuten, erhält man indem man Aminoalkylcyanide der Formel XV mit Oxalylchlorid oder -bromid umsetzt.
Synthesen dieses Typs und Pyrazinone der Formel XIIb sind in J. Het. Chem. 20, 919-923 (1983) beschrieben.

Die Pyrazinone der Unterformel XIIc

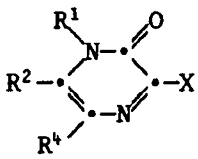

(XIIc)

worin $R^1$, $R^2$ und $R^4$ die in Formel I gegebene Bedeutung haben und X Chlor oder Brom ist, erhält man indem man Pyrazinone der Formel XVIII

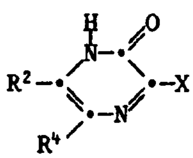

(XVIII)

worin $R^2$, $R^4$ und X die unter Formel XIIc gegebene Bedeutung haben, mit Alkylierungsmitteln wie z.B. Alkylhalogeniden, Alkyltosylaten oder Dialkylsulfaten in Gegenwart einer Base umsetzt. Die Ausgangsprodukte der Formel XVIII sind bekannt oder können analog zu publizierten Methoden erhalten werden.

Die Wirkstoffe der Formel I sind stabile Verbindungen. Ihre Handhabung bedarf keiner vorsorglichen Massnahmen.

Bei geringeren Aufwandrengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Reis, Baumwolle, Soja und Mais befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzte Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Bei grösseren Aufwandmengen an Wirkstoffen der Formel I werden alle getesteten Pflanzen in ihrer Entwicklung so geschädigt, dass sie absterben.

Die Erfindung betrifft auch herbizide und pflanzenwachstumsregulierende Mittel, welche einen neuen Wirk-

stoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazol- derivate oder Alkylarylsulfonate.

Die Fettsulfate oder -sulfonate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niederige, gegebenenfalls halogenierte Alkyl-, Benzyl-oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylaminoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1981;
H. Stache, "Tensid-Taschenbuch", 2. Aufl., C. Hanser Verlag, München, Wien, 1981;
M. and J. Ash. "Encyclopedia of Surfactants", Vol. I-III, Chemical Publishing Co., New York, 1980-1981.

Die agrochemischen Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

Emulgierbare Konzentrate:

| | |
|---|---|
| Aktiver Wirkstoff: | 1 bis 20 %, bevorzugt 5 bis 10 % |
| oberflächenaktive Mittel: | 5 bis 30 %, vorzugsweise 10 bis 20 % |
| flüssiges Trägermittel: | 50 bis 94 %, vorzugsweise 70 bis 85 % |

Stäube:

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

Suspensions-Konzentrate:

| | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

Benetzbares Pulver:

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 95 %, vorzugsweise 15 bis 90 % |

Granulate:

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiele

Beispiel H1: 3-[3-(2-Methoxycarbonylphenylsulfonyl)-ureido]-5-chlor-1-methylpyrazin-2-on. (Verbindung 1.01)

Ein Gemisch aus 2,41 g 2-Methoxycarbonylphenylsulfonylisocyanat, 1,6 g 3-Amino-5-chlor-1-methylpyrazin-2-on und 40 ml absolutem Dioxan wird für 3 Stunden zum Rückfluss erhitzt. Die Reaktionsmischung wird bis zur Trockne eingedampft und der Rückstand durch Behandlung mit Aceton kristallisiert. Man erhält so 3,8 g 3-[3-(2-Methoxycarbonylphenylsulfonyl)-ureido]-5-chlor-1-methyl-pyrazin-2-on mit einem Schmelzpunkt von 213°-214°C.

Beispiel H2: 6-[3-(2-Methoxycarbonylphenylsulfonyl)-ureido]-4-methyl-3-methylthio-1,2,4-triazin-5-on. (Verbindung 7.06)

3,42 g 6-Amino-4-methyl-3-methylthio-1,2,4-triazin-5-on werden in 100 ml absolutem Dioxan suspendiert und bei Raumtemperatur mit 4,99 g 2-Methoxycarbonylphenylsulfonylisocyanat versetzt. Die Reaktion verläuft leicht exotherm. Nach einer Reaktionszeit von einer Stunde Raumtemperatur ist die Umsetzung beendet. Der ausgefallene Niederschlag wird abfiltriert und mit einer kleinen Menge Dioxan gewaschen. Nach dem Trocknen erhält man so 6,0 g 6-[3-(2-Methoxycarbonylphenylsulfonyl)-ureido]-4-methyl-3-methylthio -1,2,4-triazin-5-on mit einem Schmelzpunkt von 186°-187°C.

Beispiel H3: 3-Amino-1,6-dimethylpyrazin-2-on. (Verbindung 14.12)

Zu einer Mischung von 8,13 g 3-Amino-6-methylpyrazin-2-on und 72 ml IN Natronlauge lässt man 8,87 g Dimethylsulfat bei Raumtemperatur zutropfen. Nachdem das Gemisch für 15 Stunden bei 20-25°C gerührt worden ist, wird der Niederschlag abfiltriert, das Filtrat auf ein Viertel des Volumens eingeengt und mit Aethylacetat extrahiert. Durch Waschen mit gesättigter Kochsalzlösung, Trocknen mit Natriumsulfat und Einengen der organischen Phase erhält man 1,7 g 3-Amino-1,6-dimethylpyrazin-2-on mit einem Schmelzpunkt von 217-219°C.

Beispiel H4: 6-Amino-4-methyl-3-methylsulfinyl-1,2,4-triazin-5-on. (Verbindung 13.25)

1,7 g 6-Amino-4-methyl-3-methylthio-1,2,4-triazin-5-on werden in 200 ml Methylenchlorid dispergiert und bei 15-20°C portionenweise mit 1,8 g 3-Chlorperbenzoesäure versetzt. Dabei entsteht eine gelbe, klare Lösung, welche man bei 40°C im Vakuum eindampft. Den festen Rückstand behandelt man mit Aethylacetat, wobei die bei der Reaktion entstandene 3-Chlorbenzoesäure vollständig in Lösung geht. Das ungelöste, reine Produkt filtriert man ab und erhält so 1,1 g 6-Amino-4-methyl-3-methylsulfinyl-1,2,4-triazin-5-on mit einem Schmelzpunkt von 189°C (Zersetzung).

Beispiel H5: 6-Amino-3-methoxy-4-methyl-1,2,4-triazin-5-on. (Verbindung 13.03)

4,1 g 6-Amino-4-methyl-3-methylsulfinyl-1,2,4-triazin-5-on werden in 50 ml absolutem Methanol suspendiert und mit 4,3 ml 30%iger methanolischer Natriummethylatlösung bei Raumtemperatur versetzt. Die Reaktion verläuft leicht exotherm und die Temperatur steigt auf 35°C. Anschliessend rührt man die Lösung für 30 Minuten bei 50°C, wobei aus der gelben, klaren Lösung ein farbloser Niederschlag ausfällt. Das Reaktionsgemisch wird eingedampft, der Rückstand mit Aethylacetat und Eiswasser aufgenommen. Die organische Phase wird abgetrennt, mit Magnesiumsulfat getrocknet und eingedampft. Der Rückstand wird mit wenig Methanol gewaschen und getrocknet. Auf diese Weise erhält man 1,6 g 6-Amino-4-methyl-3-methoxy-1,2,4-triazin-5-on mit einem Schmelzpunkt von 198-199°C.

Beispiel H6: 3-[3-(2-Chlor-pyridin-3-ylsulfonyl)ureido]-5-chlor-1-methylpyrazin-2-on. (Verbindung 5.01)

a) N-(5-Chlor-1-methylpyrazin-2-on-3-yl)-phenylcarbamat.
Zu einer Mischung von 1,9 g 55%-iger Natriumhydrid-Dispersion und 20 ml Dimethylformamid lässt man bei 10-15°C eine Lösung von 9,4 g Diphenylcarbonat und 65 ml Dimethylformamid zufliessen. Anschliessend werden 6,4 g 3-Amino-5-chlor-1-methylpyrazin-2-on bei gleicher Temperatur portionenweise eingetragen und weitere 45 Minuten gerührt. Durch Filtrieren, Eingiessen des Filtrates in ein Gemisch von 270 ml Aethylacetat, 270 ml Eiswasser und 42 ml 7%-iger Salzsäure, Waschen der organischen Phase mit Waser und Kochsalzlösung, Trocknen über Natriumsulfat, Einengen der Lösung und Abfiltrieren des ausgefallenen Produktes erhält man 6,5 g N-(5-Chlor-1-methyl-pyrazin-2-on-3-yl)-phenylcarbamat.

b) Zu einer Mischung von 1,93 g 2-Chlor-pyridin-3-yl-sulfonamid, 2,8 g N-5-Chlor-(1-methylpyrazin-2-on-3-yl)-phenylcarbamat lässt man eine Lösung von 1,52 g 1,8-Diazabicyclo-[5.4.0]-undec-7-en und 10 ml Dioxan zutropfen. Dann wird das Reaktionsgemisch für 15 Stunden bei 20-25°C gerührt. Durch Eingiessen in Wasser, Zutropfen von 10%-iger Salzsäure bis zu einem pH von 4,5, Abfiltrieren des gebildeten Niederschlags, Waschen mit Wasser und Trocknen erhält man 3,2 g 3-[3-(2-Chlor-pyridin-3-ylsulfonyl)ureido]-5-chlor-1-methylpyrazin-2-on vom Schmelzpunkt 202-203°C (Zers.).

Beispiel H7: 3-Amino-5-methoxy-1-methylpyrazin-2-on. (Verbindung 14.03)

a) N-Cyanomethyl-N-methyl-methoxalylamid.

Zu einer Mischung von 12,3 g Methoxalylchlorid, 15,8 g Methylaminoacetonitril-hydrochlorid und 80 ml Methylenchlorid lässt ran eine Lösung von 15,8 g Pyridin und 20 ml Methylenchlorid zutropfen. Anschliessend wird die Mischung für 3 Stunden bei 20-25°C gerührt. Dann wird mit 50 ml Wasser versetzt und die organische Phase nacheinander mit 5%-iger Salzsäure, Wasser und Natriumchloridlösung gewaschen. Durch Trocknen und Eindampfen erhält man 14,1 g N-Cyanomethyl-N-methyl-methoxalylamid.

b) 3-Hydroxy-5-methoxy-1-methylpyrazin-2-on.

Zu einer Lösung von 14,4 g N-Cyanomethyl-N-methyl-methoxalylamid und 200 ml absolutem Methanol lässt man 33,2 g 30%-ige methanolische Natriummethylatlösung zufliessen. Dann wird für 6 Stunden bei 55-60°C gerührt und anschliessend vollständig eingedampft. Der Rückstand wird mit Eiswasser verrührt und durch Zutropfen von 30%-iger Salzsäure auf pH 6,5 gestellt. Durch Abfiltrieren, Waschen mit Eiswasser und Trocknen erhält man 8,3 g 3-Hydroxy-5-methoxy-1-methylpyrazin-2-on vom Schmelzpunkt 201-204°C.

c) 5-Methoxy-1-methyl-3-trifluormethylsulfonyloxypyrazin-2-on.

Zu einer Suspension von 31,2 g 3-Hydroxy-5-methoxy-1-methylpyrazin-2-on, 15,8 g Pyridin und 500 ml Methylenchlorid lässt man bei 20-25°C 62 g Trifluormethansulfonsäure-anhydrid zutropfen. Die gebildete Lösung wird für weitere 2 Stunden bei 20-25°C gerührt und anschliessend mit 200 ml Eiswasser versetzt. Durch Abtrennen der organischen Phase, Waschen mit 10%-iger Natriumbicarbonatlösung und Wasser, Trocknen über Natriumsulfat und Eindampfen erhält man 39,5 g 5-Methoxy-1-methyl-3-trifluormethylsulfonyloxy-pyrazin-2-on vom Schmelzpunkt 107-108°C.

d) In eine Lösung von 28,8 g 5-Methoxy-1-methyl-3-trifluormethylsulfonyloxy-pyrazin-2-on und 100 ml Tetrahydrofuran werden bei 5 bis 10°C innerhalb von 1,5 Stunden 4,7 g Ammoniak eingeleitet. Es entsteht eine dunkle Suspension, die für 2 Stunden bei 0 bis +5°C nachgerührt wird. Durch Abfiltrieren des Niederschlags und Waschen mit kaltem Tetrahydrofuran erhält man 10,6 g 3-Amino-5-methoxy-1-methyl-pyrazin-2-on vom Schmelzpunkt 166-167°C.

Beispiel H8: 1-Aethyl-3-amino-5-brompyrazin-2-on. (Verbindung 14.16)

a) 3,5-Dibrom-pyrazin-2-on.

Zu einer Lösung von 3,45 g Natriumnitrit und 27,5 ml 96%-iger Schwefelsäure gibt man tropfenweise bei 0° bis 5°C eine Lösung von 12,65 g 2-Amino-3,5-dibrom-pyrazin und 18,5 ml 96%-iger Schwefelsäure. Anschliessend lässt man in 2 Stunden auf 20°C erwärmen und rührt für weitere 15 Stunden bei 20-25°C. Durch Eingiessen des Reaktionsgemisches in Eis, Abfiltrieren, Waschen mit Eiswasser und Trocknen erhält man 5,8 g 3,5-Dibrom-pyrazin-2-on.

b) 1-Aethyl-3,5-dibrom-pyrazin-2-on.

6,2 g 3,5-Dibrom-pyrazin-2-on werden in einem Gemisch von 38 ml Wasser, 11,1 ml 2N Natronlauge und 0,5 g Natriumcarbonat suspendiert. Bei 20-25°C werden dann tropfenweise 4,3 g Diäthylsulfat zugegeben und anschliessend wird für 15 Stunden bei gleicher Temperatur gerührt. Durch Abfiltrieren bei 0°C, Waschen mit Eiswasser und Trocknen erhält man 4,6 g 1-Aethyl-3,5-dibrom-pyrazin-2-on.

c) Eine Mischung von 4,6 g 1-Aethyl-3,5-dibrompyrazin-2-on, 9,6 ml Dioxan und 6,4 ml 30%-iger Ammoniak werden während 15 Stunden bei 20-25°C gerührt. Durch Abfiltrieren und Waschen mit Alkohol und Aether erhält man 1,3 g 1-Aethyl-3-amino-5-brom-pyrazin-2-on mit einem Schmelzpunkt 227-230°C.

In analoger Weise erhält man die in der folgenden Tabellen 1 bis 14 aufgeführten Zwischen- und Endprodukte.

Tabelle 1:

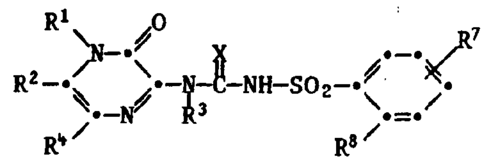

| Verb. Nr. | $R^1$ | $R^2$ | $R^3$ | $R^4$ | $R^7$ | $R^8$ | X | Smp. [°C] |
|---|---|---|---|---|---|---|---|---|
| 1.01 | $CH_3$ | H | H | Cl | H | $COOCH_3$ | O | 213-214 |
| 1.02 | $CH_3$ | H | H | Br | H | $COOCH_3$ | O | 206-208 |
| 1.03 | $CH_3$ | H | H | H | H | $COOCH_3$ | O | 208-209 |
| 1.04 | $CH_3$ | H | H | $CH_3$ | H | $COOCH_3$ | O | |
| 1.05 | $CH_3$ | H | H | $OCH_3$ | H | $COOCH_3$ | O | 172-173 |
| 1.06 | $CH_3$ | H | H | $N(CH_3)_2$ | H | $COOCH_3$ | O | |
| 1.07 | $CH_3$ | H | H | $SCH_3$ | H | $COOCH_3$ | O | |
| 1.08 | $CH_3$ | H | H | $OC_2H_5$ | H | $COOCH_3$ | O | |
| 1.09 | $CH_3$ | H | H | $-OCH_2-CF_3$ | H | $COOCH_3$ | O | |
| 1.10 | $CH_3$ | H | H | $CF_3$ | H | $COOCH_3$ | O | |
| 1.11 | $CH_3$ | $CH_3$ | H | $CH_3$ | H | $COOCH_3$ | O | 198-199 |
| 1.12 | $CH_3$ | $CH_3$ | H | H | H | $COOCH_3$ | O | 192-193 |
| 1.13 | $CH_3$ | $CH_3$ | H | Cl | H | $COOCH_3$ | O | |
| 1.14 | $CH_3$ | $CH_3$ | H | $OCH_3$ | H | $COOCH_3$ | O | |
| 1.15 | $C_2H_5$ | H | H | Cl | H | $COOCH_3$ | O | |
| 1.16 | $C_2H_5$ | H | H | Br | H | $COOCH_3$ | O | 183-186 |
| 1.17 | $C_2H_5$ | H | H | H | H | $COOCH_3$ | O | |
| 1.18 | $C_2H_5$ | H | H | $CH_3$ | H | $COOCH_3$ | O | |
| 1.19 | $C_2H_5$ | H | H | $OCH_3$ | H | $COOCH_3$ | O | |
| 1.20 | $C_2H_5$ | H | H | $N(CH_3)_2$ | H | $COOCH_3$ | O | |
| 1.21 | $C_2H_5$ | H | H | $SCH_3$ | H | $COOCH_3$ | O | |
| 1.22 | $C_2H_5$ | H | H | $OC_2H_5$ | H | $COOCH_3$ | O | |
| 1.23 | $C_2H_5$ | H | H | $-OCH_2-CF_3$ | H | $COOCH_3$ | O | |

| Verb. Nr. | $R^1$ | $R^2$ | $R^3$ | $R^4$ | $R^7$ | $R^8$ | X | Smp. [°C] |
|---|---|---|---|---|---|---|---|---|
| 1.24 | $C_2H_5$ | H | H | $CF_3$ | H | $COOCH_3$ | O | |
| 1.25 | $C_2H_5$ | $CH_3$ | H | $CH_3$ | H | $COOCH_3$ | O | |
| 1.26 | $C_2H_5$ | $CH_3$ | H | H | H | $COOCH_3$ | O | |
| 1.27 | $C_2H_5$ | $CH_3$ | H | Cl | H | $COOCH_3$ | O | |
| 1.28 | $C_2H_5$ | $CH_3$ | H | $OCH_3$ | H | $COOCH_3$ | O | |
| 1.29 | $CH_3$ | H | H | Cl | H | $-SO_2N(CH_3)_2$ | O | 225-226 |
| 1.30 | $CH_3$ | H | H | Br | H | $-SO_2N(CH_3)_2$ | O | |
| 1.31 | $CH_3$ | H | H | H | H | $-SO_2N(CH_3)_2$ | O | |
| 1.32 | $CH_3$ | H | H | $CH_3$ | H | $-SO_2N(CH_3)_2$ | O | |
| 1.33 | $CH_3$ | H | H | $OCH_3$ | H | $-SO_2N(CH_3)_2$ | O | |
| 1.34 | $CH_3$ | H | H | $N(CH_3)_2$ | H | $-SO_2N(CH_3)_2$ | O | |
| 1.35 | $CH_3$ | H | H | $SCH_3$ | H | $-SO_2N(CH_3)_2$ | O | |
| 1.36 | $CH_3$ | H | H | $OC_2H_5$ | H | $-SO_2N(CH_3)_2$ | O | |
| 1.37 | $CH_3$ | H | H | $-OCH_2-CF_3$ | H | $-SO_2N(CH_3)_2$ | O | |
| 1.38 | $CH_3$ | H | H | $CF_3$ | H | $-SO_2N(CH_3)_2$ | O | |
| 1.39 | $CH_3$ | $CH_3$ | H | $CH_3$ | H | $-SO_2N(CH_3)_2$ | O | |
| 1.40 | $CH_3$ | $CH_3$ | H | H | H | $-SO_2N(CH_3)_2$ | O | |
| 1.41 | $CH_3$ | $CH_3$ | H | Cl | H | $-SO_2N(CH_3)_2$ | O | |
| 1.42 | $CH_3$ | $CH_3$ | H | $OCH_3$ | H | $-SO_2N(CH_3)_2$ | O | |
| 1.43 | $C_2H_5$ | H | H | Cl | H | $-SO_2N(CH_3)_2$ | O | |
| 1.44 | $C_2H_5$ | H | H | Br | H | $-SO_2N(CH_3)_2$ | O | |
| 1.45 | $C_2H_5$ | H | H | H | H | $-SO_2N(CH_3)_2$ | O | |
| 1.46 | $C_2H_5$ | H | H | $CH_3$ | H | $-SO_2N(CH_3)_2$ | O | |
| 1.47 | $C_2H_5$ | H | H | $OCH_3$ | H | $-SO_2N(CH_3)_2$ | O | |
| 1.48 | $C_2H_5$ | H | H | $N(CH_3)_2$ | H | $-SO_2N(CH_3)_2$ | O | |
| 1.49 | $C_2H_5$ | H | H | $SCH_3$ | H | $-SO_2N(CH_3)_2$ | O | |
| 1.50 | $C_2H_5$ | H | H | $OC_2H_5$ | H | $-SO_2N(CH_3)_2$ | O | |
| 1.51 | $C_2H_5$ | H | H | $-OCH_2-CF_3$ | H | $-SO_2N(CH_3)_2$ | O | |
| 1.52 | $C_2H_5$ | H | H | $CF_3$ | H | $-SO_2N(CH_3)_2$ | O | |

| Verb. Nr. | $R^1$ | $R^2$ | $R^3$ | $R^4$ | $R^7$ | $R^8$ | X | Smp.[°C] |
|---|---|---|---|---|---|---|---|---|
| 1.53 | $C_2H_5$ | $CH_3$ | H | $CH_3$ | H | $-SO_2N(CH_3)_2$ | O | |
| 1.54 | $C_2H_5$ | $CH_3$ | H | H | H | $-SO_2N(CH_3)_2$ | O | |
| 1.55 | $C_2H_5$ | $CH_3$ | H | Cl | H | $-SO_2N(CH_3)_2$ | O | |
| 1.56 | $C_2H_5$ | $CH_3$ | H | $OCH_3$ | H | $-SO_2N(CH_3)_2$ | O | |
| 1.57 | $CH_3$ | H | H | Cl | H | $OC_2H_5$ | O | 237-239 |
| 1.58 | $CH_3$ | H | H | Br | H | $OC_2H_5$ | O | |
| 1.59 | $CH_3$ | H | H | H | H | $OC_2H_5$ | O | |
| 1.60 | $CH_3$ | H | H | $CH_3$ | H | $OC_2H_5$ | O | |
| 1.61 | $CH_3$ | H | H | $OCH_3$ | H | $OC_2H_5$ | O | 197-199 |
| 1.62 | $CH_3$ | H | H | $N(CH_3)_2$ | H | $OC_2H_5$ | O | |
| 1.63 | $CH_3$ | H | H | $SCH_3$ | H | $OC_2H_5$ | O | |
| 1.64 | $CH_3$ | H | H | $OC_2H_5$ | H | $OC_2H_5$ | O | |
| 1.65 | $CH_3$ | H | H | $-OCH_2-CF_3$ | H | $OC_2H_5$ | O | |
| 1.66 | $CH_3$ | H | H | $CF_3$ | H | $OC_2H_5$ | O | |
| 1.67 | $CH_3$ | $CH_3$ | H | $CH_3$ | H | $OC_2H_5$ | O | |
| 1.68 | $CH_3$ | $CH_3$ | H | H | H | $OC_2H_5$ | O | |
| 1.69 | $CH_3$ | $CH_3$ | H | Cl | H | $OC_2H_5$ | O | |
| 1.70 | $CH_3$ | $CH_3$ | H | $OCH_3$ | H | $OC_2H_5$ | O | |
| 1.71 | $C_2H_5$ | H | H | Cl | H | $OC_2H_5$ | O | |
| 1.72 | $C_2H_5$ | H | H | Br | H | $OC_2H_5$ | O | |
| 1.73 | $C_2H_5$ | H | H | H | H | $OC_2H_5$ | O | |
| 1.74 | $C_2H_5$ | H | H | $CH_3$ | H | $OC_2H_5$ | O | |
| 1.75 | $C_2H_5$ | H | H | $OCH_3$ | H | $OC_2H_5$ | O | |
| 1.76 | $C_2H_5$ | H | H | $N(CH_3)_2$ | H | $OC_2H_5$ | O | |
| 1.77 | $C_2H_5$ | H | H | $SCH_3$ | H | $OC_2H_5$ | O | |
| 1.78 | $C_2H_5$ | H | H | $OC_2H_5$ | H | $OC_2H_5$ | O | |
| 1.79 | $C_2H_5$ | H | H | $-OCH_2-CF_3$ | H | $OC_2H_5$ | O | |
| 1.80 | $C_2H_5$ | H | H | $CF_3$ | H | $OC_2H_5$ | O | |
| 1.81 | $C_2H_5$ | $CH_3$ | H | $CH_3$ | H | $OC_2H_5$ | O | |

| Verb. Nr. | $R^1$ | $R^2$ | $R^3$ | $R^4$ | $R^7$ | $R^8$ | X | Smp.[°C] |
|---|---|---|---|---|---|---|---|---|
| 1.82 | $C_2H_5$ | $CH_3$ | H | H | H | $OC_2H_5$ | O | |
| 1.83 | $C_2H_5$ | $CH_3$ | H | Cl | H | $OC_2H_5$ | O | |
| 1.84 | $C_2H_5$ | $CH_3$ | H | $OCH_3$ | H | $OC_2H_5$ | O | |
| 1.85 | $CH_3$ | H | H | Cl | H | $OCHF_2$ | O | 204-205 |
| 1.86 | $CH_3$ | H | H | Br | H | $OCHF_2$ | O | |
| 1.87 | $CH_3$ | H | H | H | H | $OCHF_2$ | O | |
| 1.88 | $CH_3$ | H | H | $CH_3$ | H | $OCHF_2$ | O | |
| 1.89 | $CH_3$ | H | H | $OCH_3$ | H | $OCHF_2$ | O | |
| 1.90 | $CH_3$ | H | H | $N(CH_3)_2$ | H | $OCHF_2$ | O | 171-172 |
| 1.91 | $CH_3$ | H | H | $SCH_3$ | H | $OCHF_2$ | O | |
| 1.92 | $CH_3$ | H | H | $OC_2H_5$ | H | $OCHF_2$ | O | |
| 1.93 | $CH_3$ | H | H | $-OCH_2-CF_3$ | H | $OCHF_2$ | O | |
| 1.94 | $CH_3$ | H | H | $CF_3$ | H | $OCHF_2$ | O | |
| 1.95 | $CH_3$ | $CH_3$ | H | $CH_3$ | H | $OCHF_2$ | O | |
| 1.96 | $CH_3$ | $CH_3$ | H | H | H | $OCHF_2$ | O | |
| 1.97 | $CH_3$ | $CH_3$ | H | Cl | H | $OCHF_2$ | O | |
| 1.98 | $CH_3$ | $CH_3$ | H | $OCH_3$ | H | $OCHF_2$ | O | |
| 1.99 | $C_2H_5$ | H | H | Cl | H | $OCHF_2$ | O | |
| 1.100 | $C_2H_5$ | H | H | Br | H | $OCHF_2$ | O | |
| 1.101 | $C_2H_5$ | H | H | H | H | $OCHF_2$ | O | |
| 1.102 | $C_2H_5$ | H | H | $CH_3$ | H | $OCHF_2$ | O | |
| 1.103 | $C_2H_5$ | H | H | $OCH_3$ | H | $OCHF_2$ | O | |
| 1.104 | $C_2H_5$ | H | H | $N(CH_3)_2$ | H | $OCHF_2$ | O | |
| 1.105 | $C_2H_5$ | H | H | $SCH_3$ | H | $OCHF_2$ | O | |
| 1.106 | $C_2H_5$ | H | H | $OC_2H_5$ | H | $OCHF_2$ | O | |
| 1.107 | $C_2H_5$ | H | H | $-OCH_2-CF_3$ | H | $OCHF_2$ | O | |
| 1.108 | $C_2H_5$ | H | H | $CF_3$ | H | $OCHF_2$ | O | |
| 1.109 | $C_2H_5$ | $CH_3$ | H | $CH_3$ | H | $OCHF_2$ | O | |
| 1.110 | $C_2H_5$ | $CH_3$ | H | H | H | $OCHF_2$ | O | |

| Verb. Nr. | $R^1$ | $R^2$ | $R^3$ | $R^4$ | $R^7$ | $R^8$ | X | Smp. [°C] |
|---|---|---|---|---|---|---|---|---|
| 1.111 | $C_2H_5$ | $CH_3$ | H | Cl | H | $OCHF_2$ | O | |
| 1.112 | $C_2H_5$ | $CH_3$ | H | $OCH_3$ | H | $OCHF_2$ | O | |
| 1.113 | $CH_3$ | H | H | Cl | H | $CF_3$ | O | |
| 1.114 | $CH_3$ | H | H | Br | H | $CF_3$ | O | |
| 1.115 | $CH_3$ | H | H | H | H | $CF_3$ | O | |
| 1.116 | $CH_3$ | H | H | $CH_3$ | H | $CF_3$ | O | |
| 1.117 | $CH_3$ | H | H | $OCH_3$ | H | $CF_3$ | O | |
| 1.118 | $CH_3$ | H | H | $N(CH_3)_2$ | H | $CF_3$ | O | |
| 1.119 | $CH_3$ | H | H | $SCH_3$ | H | $CF_3$ | O | |
| 1.120 | $CH_3$ | H | H | $OC_2H_5$ | H | $CF_3$ | O | |
| 1.121 | $CH_3$ | H | H | $-OCH_2-CF_3$ | H | $CF_3$ | O | |
| 1.122 | $CH_3$ | H | H | $CF_3$ | H | $CF_3$ | O | |
| 1.123 | $CH_3$ | $CH_3$ | H | $CH_3$ | H | $CF_3$ | O | |
| 1.124 | $CH_3$ | $CH_3$ | H | H | H | $CF_3$ | O | |
| 1.125 | $CH_3$ | $CH_3$ | H | Cl | H | $CF_3$ | O | |
| 1.126 | $CH_3$ | $CH_3$ | H | $OCH_3$ | H | $CF_3$ | O | |
| 1.127 | $C_2H_5$ | H | H | Cl | H | $CF_3$ | O | |
| 1.128 | $C_2H_5$ | H | H | Br | H | $CF_3$ | O | |
| 1.129 | $C_2H_5$ | H | H | H | H | $CF_3$ | O | |
| 1.130 | $C_2H_5$ | H | H | $CH_3$ | H | $CF_3$ | O | |
| 1.131 | $C_2H_5$ | H | H | $OCH_3$ | H | $CF_3$ | O | |
| 1.132 | $C_2H_5$ | H | H | $N(CH_3)_2$ | H | $CF_3$ | O | |
| 1.133 | $C_2H_5$ | H | H | $SCH_3$ | H | $CF_3$ | O | |
| 1.134 | $C_2H_5$ | H | H | $OC_2H_5$ | H | $CF_3$ | O | |
| 1.135 | $C_2H_5$ | H | H | $-OCH_2-CF_3$ | H | $CF_3$ | O | |
| 1.136 | $C_2H_5$ | H | H | $CF_3$ | H | $CF_3$ | O | |
| 1.137 | $C_2H_5$ | H | H | $CH_3$ | H | $CF_3$ | O | |
| 1.138 | $C_2H_5$ | H | H | H | H | $CF_3$ | O | |
| 1.139 | $C_2H_5$ | H | H | Cl | H | $CF_3$ | O | |

| Verb. Nr. | $R^1$ | $R^2$ | $R^3$ | $R^4$ | $R^7$ | $R^8$ | X | Smp. [°C] |
|---|---|---|---|---|---|---|---|---|
| 1.140 | $C_2H_5$ | H | H | $OCH_3$ | H | $CF_3$ | O | |
| 1.141 | $C_3H_7$-i | H | H | Cl | H | $COOCH_3$ | O | |
| 1.142 | $C_3H_7$-i | H | H | $CH_3$ | H | $COOCH_3$ | O | |
| 1.143 | $C_3H_7$-i | H | H | $OCH_3$ | H | $COOCH_3$ | O | |
| 1.144 | $CH_3$ | H | H | Cl | H | $COOCH_3$ | S | |
| 1.145 | $CH_3$ | H | $CH_3$ | Cl | H | $COOCH_3$ | O | |
| 1.146 | $CH_3$ | H | $C_2H_5$ | Cl | H | $COOCH_3$ | O | |
| 1.147 | $CH_3$ | H | H | Cl | 5-$OCH_3$ | $COOCH_3$ | O | |
| 1.148 | $CH_3$ | H | H | Cl | 5-F | $COOCH_3$ | O | |
| 1.149 | $CH_3$ | H | H | Cl | 6-Cl | $COOCH_3$ | O | |
| 1.150 | $C_3H_7$-i | H | H | Cl | H | $OCHF_2$ | O | |
| 1.151 | $C_3H_7$-i | H | H | $CH_3$ | H | $OCHF_2$ | O | |
| 1.152 | $C_3H_7$-i | H | H | $OCH_3$ | H | $OCHF_2$ | O | |
| 1.153 | $CH_3$ | H | $CH_3$ | Cl | H | $OCHF_2$ | O | |
| 1.154 | $CH_3$ | H | $C_2H_5$ | Cl | H | $OCHF_2$ | O | |
| 1.155 | $CH_3$ | H | H | Cl | 5-$OCH_3$ | $OCHF_2$ | O | |
| 1.156 | $CH_3$ | H | H | Cl | 5-F | $OCHF_2$ | O | |
| 1.157 | $CH_3$ | H | H | Cl | 5-Cl | $OCHF_2$ | O | |
| 1.158 | $CH_3$ | H | H | Cl | H | $CHF_2$ | O | |
| 1.159 | $CH_3$ | H | H | $OCH_3$ | H | $CHF_2$ | O | |
| 1.160 | $CH_3$ | H | H | $OC_2H_5$ | H | $CHF_2$ | O | |
| 1.161 | $CH_3$ | H | H | Cl | H | $OCH_3$ | O | |
| 1.162 | $CH_3$ | H | H | $OCH_3$ | H | $OCH_3$ | O | |
| 1.163 | $CH_3$ | H | H | $OC_2H_5$ | H | $OCH_3$ | O | |
| 1.164 | $CH_3$ | H | H | Cl | H | $-OCH_2-CH_2Cl$ | O | 238-239 |
| 1.165 | $CH_3$ | H | H | $OCH_3$ | H | $-OCH_2-CH_2Cl$ | O | |
| 1.166 | $CH_3$ | H | H | $OC_2H_5$ | H | $-OCH_2-CH_2Cl$ | O | |
| 1.167 | $CH_3$ | H | H | Cl | H | $-OCH_2-CH_2OCH_3$ | O | |
| 1.168 | $CH_3$ | H | H | $OCH_3$ | H | $-OCH_2-CH_2OCH_3$ | O | |

| Verb. Nr. | $R^1$ | $R^2$ | $R^3$ | $R^4$ | $R^7$ | $R^8$ | X | Smp. [°C] |
|---|---|---|---|---|---|---|---|---|
| 1.169 | $CH_3$ | H | H | $OC_2H_5$ | H | $-OCH_2-CH_2OCH_3$ | O | |
| 1.170 | $CH_3$ | H | H | Cl | H | $-OCH_2-CF_3$ | O | |
| 1.171 | $CH_3$ | H | H | $OCH_3$ | H | -O-CCl=CHCl | O | |
| 1.172 | $CH_3$ | H | H | $OC_2H_5$ | H | -O-CCl=CHCl | O | |
| 1.173 | $CH_3$ | H | H | Cl | H | -O-CCl=CHCl | O | |
| 1.174 | $CH_3$ | H | H | $OC_2H_5$ | H | $-OCH_2-CF_3$ | O | |
| 1.175 | $CH_3$ | H | H | $OCH_3$ | H | $-OCH_2-CF_3$ | O | |
| 1.176 | $CH_3$ | H | H | Cl | H | $NO_2$ | O | |
| 1.177 | $CH_3$ | H | H | $OCH_3$ | H | $NO_2$ | O | |
| 1.178 | $CH_3$ | H | H | $OC_2H_5$ | H | $NO_2$ | O | |
| 1.179 | $CH_3$ | H | H | Cl | H | $COOC_2H_5$ | O | |
| 1.180 | $CH_3$ | H | H | $OCH_3$ | H | $COOC_2H_5$ | O | |
| 1.181 | $CH_3$ | H | H | $OC_2H_5$ | H | $COOC_2H_5$ | O | |
| 1.182 | $CH_3$ | H | H | Cl | H | Cl | O | |
| 1.183 | $CH_3$ | H | H | $OCH_3$ | H | Cl | O | |
| 1.184 | $CH_3$ | H | H | $OC_2H_5$ | H | Cl | O | |
| 1.185 | $CH_3$ | H | H | Cl | H | F | O | |
| 1.186 | $CH_3$ | H | H | $OCH_3$ | H | F | O | |
| 1.187 | $CH_3$ | H | H | $OC_2H_5$ | H | F | O | |
| 1.188 | $CH_3$ | H | H | Cl | H | Br | O | |
| 1.189 | $CH_3$ | H | H | $OCH_3$ | H | Br | O | |
| 1.190 | $CH_3$ | H | H | $OC_2H_5$ | H | Br | O | |
| 1.191 | $CH_3$ | H | H | Cl | H | $CH_3$ | O | |
| 1.192 | $CH_3$ | H | H | $OCH_3$ | H | $CH_3$ | O | |
| 1.193 | $CH_3$ | H | H | $OC_2H_5$ | H | $CH_3$ | O | |
| 1.194 | $CH_3$ | H | H | Cl | H | $SCH_3$ | O | |
| 1.195 | $CH_3$ | H | H | $OCH_3$ | H | $SCH_3$ | O | |
| 1.196 | $CH_3$ | H | H | $OC_2H_5$ | H | $SCH_3$ | O | |
| 1.197 | $CH_3$ | H | H | Cl | H | $SCHF_2$ | O | |

| Verb. Nr. | $R^1$ | $R^2$ | $R^3$ | $R^4$ | $R^7$ | $R^8$ | X | Smp.[°C] |
|---|---|---|---|---|---|---|---|---|
| 1.198 | $CH_3$ | H | H | $OCH_3$ | H | $SCHF_2$ | O | |
| 1.199 | $CH_3$ | H | H | $OC_2H_5$ | H | $SCHF_2$ | O | |
| 1.200 | $CH_3$ | H | H | Cl | H | $-S-CH_2-CH_2Cl$ | O | |
| 1.201 | $CH_3$ | H | H | $OCH_3$ | H | $-S-CH_2-CH_2Cl$ | O | |
| 1.202 | $CH_3$ | H | H | $OC_2H_5$ | H | $-S-CH_2-CH_2Cl$ | O | |
| 1.203 | $CH_3$ | H | H | Cl | H | $-SO_2-CH_3$ | O | |
| 1.204 | $CH_3$ | H | H | $OCH_3$ | H | $-SO_2-CH_3$ | O | |
| 1.205 | $CH_3$ | H | H | $OC_2H_5$ | H | $-SO_2-CH_3$ | O | |
| 1.206 | $CH_3$ | H | H | Cl | H | $-SO_2-C_2H_5$ | O | |
| 1.207 | $CH_3$ | H | H | $OCH_3$ | H | $-SO_2-C_2H_5$ | O | |
| 1.208 | $CH_3$ | H | H | $OC_2H_5$ | H | $-SO_2-C_2H_5$ | O | |
| 1.209 | $CH_3$ | H | H | Cl | H | $-OCH_2-CH=CH_2$ | O | |
| 1.210 | $CH_3$ | H | H | $OCH_3$ | H | $-OCH_2-CH=CH_2$ | O | |
| 1.211 | $CH_3$ | H | H | $OC_2H_5$ | H | $-OCH_2-CH=CH_2$ | O | |
| 1.212 | $CH_3$ | H | H | Cl | H | H | O | |
| 1.213 | $CH_3$ | H | H | $OCH_3$ | H | H | O | |
| 1.214 | $CH_3$ | H | H | $OC_2H_5$ | H | H | O | |
| 1.215 | $CH_3$ | H | H | Cl | H | $-O-SO_2CH_3$ | O | 239-240 (Zers.) |
| 1.216 | $CH_3$ | H | H | $OCH_3$ | H | $-O-SO_2CH_3$ | O | |
| 1.217 | $CH_3$ | H | H | $OC_2H_5$ | H | $-O-SO_2CH_3$ | O | |
| 1.218 | $CH_3$ | H | H | $OCH_3$ | H | $-OCH_2CH_2Cl$ | O | 199-200 |
| 1.219 | $CH_3$ | $OCH_3$ | H | H | H | $-COOCH_3$ | O | |
| 1.220 | $CH_3$ | $OCH_3$ | H | H | H | $-OCHF_2$ | O | |
| 1.221 | $CH_3$ | $OCH_3$ | H | Br | H | $-COOCH_3$ | O | |
| 1.222 | $CH_3$ | Cl | H | Cl | H | $-COOCH_3$ | O | |
| 1.223 | $CH_3$ | $C_2H_5$ | H | H | H | $-COOCH_3$ | O | |
| 1.224 | $CH_3$ | $C_3H_7$ | H | H | H | $-COOCH_3$ | O | |

| Verb. Nr. | $R^1$ | $R^2$ | $R^3$ | $R^4$ | $R^7$ | $R^8$ | X | Smp. [°C] |
|---|---|---|---|---|---|---|---|---|
| 1.225 | $CH_3$ | $OC_2H_5$ | H | H | H | $-COOCH_3$ | O | |
| 1.226 | $CH_3$ | $SCH_3$ | H | H | H | $-COOCH_3$ | O | |
| 1.227 | $CH_3$ | $N(CH_3)_2$ | H | H | H | $-COOCH_3$ | O | |
| 1.228 | $CH_3$ | H | H | $-CH_2OCH_3$ | H | $-COOCH_3$ | O | |
| 1.229 | $CH_3$ | H | H | $-O-C_3H_7$-i | H | $-COOCH_3$ | O | |
| 1.230 | $CH_3$ | H | H | $-C_3H_5$-cycl. | H | $-COOCH_3$ | O | |
| 1.231 | $CH_3$ | H | H | Cl | H | $-OC_3H_7$ | O | |
| 1.232 | $CH_3$ | H | H | $OCH_3$ | H | $-OC_3H_7$ | O | |
| 1.233 | $CH_3$ | H | H | $OC_2H_5$ | H | $-OC_3H_7$ | O | |

Tabelle 2:

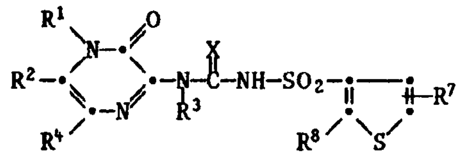

| Verb. Nr. | $R^1$ | $R^2$ | $R^3$ | $R^4$ | $R^7$ | $R^8$ | X | Smp. [°C] |
|---|---|---|---|---|---|---|---|---|
| 2.01 | $CH_3$ | H | H | Cl | H | $COOCH_3$ | O | |
| 2.02 | $CH_3$ | H | H | Br | H | $COOCH_3$ | O | |
| 2.03 | $CH_3$ | H | H | H | H | $COOCH_3$ | O | |
| 2.04 | $CH_3$ | H | H | $CH_3$ | H | $COOCH_3$ | O | |
| 2.05 | $CH_3$ | H | H | $OCH_3$ | H | $COOCH_3$ | O | |
| 2.06 | $CH_3$ | H | H | $N(CH_3)_2$ | H | $COOCH_3$ | O | |
| 2.07 | $CH_3$ | H | H | $SCH_3$ | H | $COOCH_3$ | O | |
| 2.08 | $CH_3$ | H | H | $OC_2H_5$ | H | $COOCH_3$ | O | |
| 2.09 | $CH_3$ | H | H | $-OCH_2-CF_3$ | H | $COOCH_3$ | O | |
| 2.10 | $CH_3$ | H | H | $CF_3$ | H | $COOCH_3$ | O | |
| 2.11 | $CH_3$ | $CH_3$ | H | $CH_3$ | H | $COOCH_3$ | O | |
| 2.12 | $CH_3$ | $CH_3$ | H | H | H | $COOCH_3$ | O | |
| 2.13 | $CH_3$ | $CH_3$ | H | Cl | H | $COOCH_3$ | O | |
| 2.14 | $CH_3$ | $CH_3$ | H | $OCH_3$ | H | $COOCH_3$ | O | |
| 2.15 | $C_2H_5$ | H | H | Cl | H | $COOCH_3$ | O | |
| 2.16 | $C_2H_5$ | H | H | Br | H | $COOCH_3$ | O | |
| 2.17 | $C_2H_5$ | H | H | H | H | $COOCH_3$ | O | |
| 2.18 | $C_2H_5$ | H | H | $CH_3$ | H | $COOCH_3$ | O | |
| 2.19 | $C_2H_5$ | H | H | $OCH_3$ | H | $COOCH_3$ | O | |
| 2.20 | $C_2H_5$ | H | H | $N(CH_3)_2$ | H | $COOCH_3$ | O | |
| 2.21 | $C_2H_5$ | H | H | $SCH_3$ | H | $COOCH_3$ | O | |
| 2.22 | $C_2H_5$ | H | H | $OC_2H_5$ | H | $COOCH_3$ | O | |
| 2.23 | $C_2H_5$ | H | H | $-OCH_2-CF_3$ | H | $COOCH_3$ | O | |

| Verb. Nr. | $R^1$ | $R^2$ | $R^3$ | $R^4$ | $R^7$ | $R^8$ | X | Smp. [°C] |
|---|---|---|---|---|---|---|---|---|
| 2.24 | $C_2H_5$ | H | H | $CF_3$ | H | $COOCH_3$ | O | |
| 2.25 | $C_2H_5$ | $CH_3$ | H | $CH_3$ | H | $COOCH_3$ | O | |
| 2.26 | $C_2H_5$ | $CH_3$ | H | H | H | $COOCH_3$ | O | |
| 2.27 | $C_2H_5$ | $CH_3$ | H | Cl | H | $COOCH_3$ | O | |
| 2.28 | $C_2H_5$ | $CH_3$ | H | $OCH_3$ | H | $COOCH_3$ | O | |
| 2.29 | $CH_3$ | H | H | $OC_2H_5$ | H | H | O | |
| 2.30 | $CH_3$ | H | H | Cl | H | H | O | |
| 2.31 | $CH_3$ | H | H | $OCH_3$ | H | H | O | |
| 2.32 | $CH_3$ | H | H | $OC_2H_5$ | H | Cl | O | |
| 2.33 | $CH_3$ | H | H | Cl | H | Cl | O | |
| 2.34 | $CH_3$ | H | H | $OCH_3$ | H | Cl | O | |

Tabelle 3:

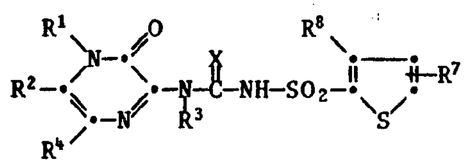

| Verb. Nr. | $R^1$ | $R^2$ | $R^3$ | $R^4$ | $R^7$ | $R^8$ | X | Smp.[°C] |
|---|---|---|---|---|---|---|---|---|
| 3.01 | $CH_3$ | H | H | Cl | H | $COOCH_3$ | O | |
| 3.02 | $CH_3$ | H | H | Br | H | $COOCH_3$ | O | |
| 3.03 | $CH_3$ | H | H | H | H | $COOCH_3$ | O | |
| 3.04 | $CH_3$ | H | H | $CH_3$ | H | $COOCH_3$ | O | |
| 3.05 | $CH_3$ | H | H | $OCH_3$ | H | $COOCH_3$ | O | |
| 3.06 | $CH_3$ | H | H | $OC_2H_5$ | H | $COOCH_3$ | O | |
| 3.07 | $CH_3$ | H | H | $SCH_3$ | H | $COOCH_3$ | O | |
| 3.08 | $CH_3$ | H | H | $N(CH_3)_2$ | H | $COOCH_3$ | O | |
| 3.09 | $CH_3$ | H | H | $-OCH_2-CF_3$ | H | $COOCH_3$ | O | |
| 3.10 | $CH_3$ | H | H | $CF_3$ | H | $COOCH_3$ | O | |
| 3.11 | $CH_3$ | $CH_3$ | H | $CH_3$ | H | $COOCH_3$ | O | |
| 3.12 | $CH_3$ | $CH_3$ | H | H | H | $COOCH_3$ | O | |
| 3.13 | $CH_3$ | $CH_3$ | H | Cl | H | $COOCH_3$ | O | |
| 3.14 | $CH_3$ | $CH_3$ | H | $OCH_3$ | H | $COOCH_3$ | O | |
| 3.15 | $C_2H_5$ | H | H | Cl | H | $COOCH_3$ | O | |
| 3.16 | $C_2H_5$ | H | H | Br | H | $COOCH_3$ | O | |
| 3.17 | $C_2H_5$ | H | H | H | H | $COOCH_3$ | O | |
| 3.18 | $C_2H_5$ | H | H | $CH_3$ | H | $COOCH_3$ | O | |
| 3.19 | $C_2H_5$ | H | H | $OCH_3$ | H | $COOCH_3$ | O | |
| 3.20 | $C_2H_5$ | H | H | $N(CH_3)_2$ | H | $COOCH_3$ | O | |
| 3.21 | $C_2H_5$ | H | H | $SCH_3$ | H | $COOCH_3$ | O | |
| 3.22 | $C_2H_5$ | H | H | $OC_2H_5$ | H | $COOCH_3$ | O | |
| 3.23 | $C_2H_5$ | H | H | $-OCH_2-CF_3$ | H | $COOCH_3$ | O | |

| Verb. Nr. | $R^1$ | $R^2$ | $R^3$ | $R^4$ | $R^7$ | $R^8$ | X | Smp.[°C] |
|---|---|---|---|---|---|---|---|---|
| 3.24 | $C_2H_5$ | H | H | $CF_3$ | H | $COOCH_3$ | O | |
| 3.25 | $C_2H_5$ | $CH_3$ | H | $CH_3$ | H | $COOCH_3$ | O | |
| 3.26 | $C_2H_5$ | $CH_3$ | H | H | H | $COOCH_3$ | O | |
| 3.27 | $C_2H_5$ | $CH_3$ | H | Cl | H | $COOCH_3$ | O | |
| 3.28 | $C_2H_5$ | $CH_3$ | H | $OCH_3$ | H | $COOCH_3$ | O | |
| 3.29 | $CH_3$ | H | H | $OC_2H_5$ | H | H | O | |
| 3.30 | $CH_3$ | H | H | Cl | H | H | O | |
| 3.31 | $CH_3$ | H | H | $OCH_3$ | H | H | O | |
| 3.32 | $CH_3$ | H | H | $OC_2H_5$ | H | Cl | O | |
| 3.33 | $CH_3$ | H | H | Cl | H | Cl | O | |
| 3.34 | $CH_3$ | H | H | $OCH_3$ | H | Cl | O | |

Tabelle 4:

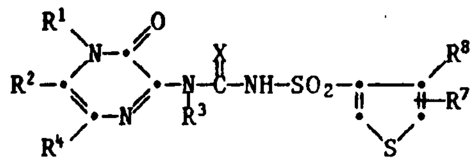

| Verb. Nr. | $R^1$ | $R^2$ | $R^3$ | $R^4$ | $R^7$ | $R^8$ | X | Smp. [°C] |
|---|---|---|---|---|---|---|---|---|
| 4.01 | $CH_3$ | H | H | Cl | H | $COOCH_3$ | O | 206-207 |
| 4.02 | $CH_3$ | H | H | Br | H | $COOCH_3$ | O | |
| 4.03 | $CH_3$ | H | H | H | H | $COOCH_3$ | O | |
| 4.04 | $CH_3$ | H | H | $CH_3$ | H | $COOCH_3$ | O | |
| 4.05 | $CH_3$ | H | H | $OCH_3$ | H | $COOCH_3$ | O | |
| 4.06 | $CH_3$ | H | H | $OC_2H_5$ | H | $COOCH_3$ | O | |
| 4.07 | $CH_3$ | H | H | $SCH_3$ | H | $COOCH_3$ | O | |
| 4.08 | $CH_3$ | H | H | $N(CH_3)_2$ | H | $COOCH_3$ | O | |
| 4.09 | $CH_3$ | H | H | $-OCH_2-CF_3$ | H | $COOCH_3$ | O | |
| 4.10 | $CH_3$ | H | H | $CF_3$ | H | $COOCH_3$ | O | |
| 4.11 | $CH_3$ | $CH_3$ | H | $CH_3$ | H | $COOCH_3$ | O | |
| 4.12 | $CH_3$ | $CH_3$ | H | H | H | $COOCH_3$ | O | |
| 4.13 | $CH_3$ | $CH_3$ | H | Cl | H | $COOCH_3$ | O | |
| 4.14 | $CH_3$ | $CH_3$ | H | $OCH_3$ | H | $COOCH_3$ | O | |
| 4.15 | $C_2H_5$ | H | H | Cl | H | $COOCH_3$ | O | |
| 4.16 | $C_2H_5$ | H | H | Br | H | $COOCH_3$ | O | |
| 4.17 | $C_2H_5$ | H | H | H | H | $COOCH_3$ | O | |
| 4.18 | $C_2H_5$ | H | H | $CH_3$ | H | $COOCH_3$ | O | |
| 4.19 | $C_2H_5$ | H | H | $OCH_3$ | H | $COOCH_3$ | O | |
| 4.20 | $C_2H_5$ | H | H | $N(CH_3)_2$ | H | $COOCH_3$ | O | |
| 4.21 | $C_2H_5$ | H | H | $SCH_3$ | H | $COOCH_3$ | O | |
| 4.22 | $C_2H_5$ | H | H | $OC_2H_5$ | H | $COOCH_3$ | O | |
| 4.23 | $C_2H_5$ | H | H | $-OCH_2-CF_3$ | H | $COOCH_3$ | O | |

| Verb. Nr. | $R^1$ | $R^2$ | $R^3$ | $R^4$ | $R^7$ | $R^8$ | X | Smp. [°C] |
|---|---|---|---|---|---|---|---|---|
| 4.24 | $C_2H_5$ | H | H | $CF_3$ | H | $COOCH_3$ | O | |
| 4.25 | $C_2H_5$ | $CH_3$ | H | $CH_3$ | H | $COOCH_3$ | O | |
| 4.26 | $C_2H_5$ | $CH_3$ | H | H | H | $COOCH_3$ | O | |
| 4.27 | $C_2H_5$ | $CH_3$ | H | Cl | H | $COOCH_3$ | O | |
| 4.28 | $C_2H_5$ | $CH_3$ | H | $OCH_3$ | H | $COOCH_3$ | O | |
| 4.29 | $CH_3$ | H | H | $OC_2H_5$ | H | H | O | |
| 4.30 | $CH_3$ | H | H | Cl | H | H | O | |
| 4.31 | $CH_3$ | H | H | $OCH_3$ | H | H | O | |
| 4.32 | $CH_3$ | H | H | $OC_2H_5$ | H | Cl | O | |
| 4.33 | $CH_3$ | H | H | Cl | H | Cl | O | |
| 4.34 | $CH_3$ | H | H | $OCH_3$ | H | Cl | O | |

Tabelle 5:

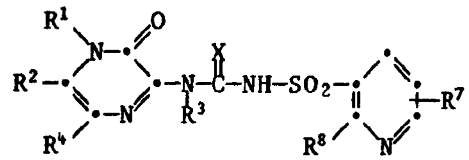

| Verb. Nr. | $R^1$ | $R^2$ | $R^3$ | $R^4$ | $R^7$ | $R^8$ | X | Smp.[°C] |
|---|---|---|---|---|---|---|---|---|
| 5.01 | $CH_3$ | H | H | Cl | H | Cl | O | 202-203 (Zers.) |
| 5.02 | $CH_3$ | H | H | Br | H | Cl | O | |
| 5.03 | $CH_3$ | H | H | H | H | Cl | O | |
| 5.04 | $CH_3$ | H | H | $CH_3$ | H | Cl | O | |
| 5.05 | $CH_3$ | H | H | $OCH_3$ | H | Cl | O | |
| 5.06 | $CH_3$ | H | H | $OC_2H_5$ | H | Cl | O | |
| 5.07 | $CH_3$ | H | H | $SCH_3$ | H | Cl | O | |
| 5.08 | $CH_3$ | H | H | $N(CH_3)_2$ | H | Cl | O | |
| 5.09 | $CH_3$ | H | H | $-OCH_2-CF_3$ | H | Cl | O | |
| 5.10 | $CH_3$ | H | H | $CF_3$ | H | Cl | O | |
| 5.11 | $CH_3$ | $CH_3$ | H | $CH_3$ | H | Cl | O | |
| 5.12 | $CH_3$ | $CH_3$ | H | H | H | Cl | O | |
| 5.13 | $CH_3$ | $CH_3$ | H | Cl | H | Cl | O | |
| 5.14 | $CH_3$ | $CH_3$ | H | $OCH_3$ | H | Cl | O | |
| 5.15 | $C_2H_5$ | H | H | Cl | H | Cl | O | |
| 5.16 | $C_2H_5$ | H | H | Br | H | Cl | O | |
| 5.17 | $C_2H_5$ | H | H | H | H | Cl | O | |
| 5.18 | $C_2H_5$ | H | H | $CH_3$ | H | Cl | O | |
| 5.19 | $C_2H_5$ | H | H | $OCH_3$ | H | Cl | O | |
| 5.20 | $C_2H_5$ | H | H | $N(CH_3)_2$ | H | Cl | O | |
| 5.21 | $C_2H_5$ | H | H | $SCH_3$ | H | Cl | O | |
| 5.22 | $C_2H_5$ | H | H | $OC_2H_5$ | H | Cl | O | |
| 5.23 | $C_2H_5$ | H | H | $-OCH_2-CF_3$ | H | Cl | O | |

| Verb. Nr. | $R^1$ | $R^2$ | $R^3$ | $R^4$ | $R^7$ | $R^8$ | X | Smp.[°C] |
|---|---|---|---|---|---|---|---|---|
| 5.24 | $C_2H_5$ | H | H | $CF_3$ | H | Cl | O | |
| 5.25 | $C_2H_5$ | $CH_3$ | H | $CH_3$ | H | Cl | O | |
| 5.26 | $C_2H_5$ | $CH_3$ | H | H | H | Cl | O | |
| 5.27 | $C_2H_5$ | $CH_3$ | H | Cl | H | Cl | O | |
| 5.28 | $C_2H_5$ | $CH_3$ | H | $OCH_3$ | H | Cl | O | |
| 5.29 | $CH_3$ | H | H | $OC_2H_5$ | H | $COOCH_3$ | O | |
| 5.30 | $CH_3$ | H | H | Cl | H | $COOCH_3$ | O | |
| 5.31 | $CH_3$ | H | H | $OCH_3$ | H | $COOCH_3$ | O | |
| 5.32 | $CH_3$ | H | H | $OC_2H_5$ | H | $OCH_3$ | O | |
| 5.33 | $CH_3$ | H | H | Cl | H | $OCH_3$ | O | |
| 5.34 | $CH_3$ | H | H | $OCH_3$ | H | $OCH_3$ | O | |

**Tabelle 6:**

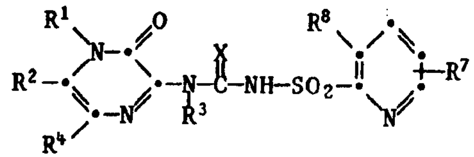

| Verb. Nr. | $R^1$ | $R^2$ | $R^3$ | $R^4$ | $R^7$ | $R^8$ | X | Smp. [°C] |
|---|---|---|---|---|---|---|---|---|
| 6.01 | $CH_3$ | H | H | Cl | H | Cl | O | |
| 6.02 | $CH_3$ | H | H | Br | H | Cl | O | |
| 6.03 | $CH_3$ | H | H | H | H | Cl | O | |
| 6.04 | $CH_3$ | H | H | $CH_3$ | H | Cl | O | |
| 6.05 | $CH_3$ | H | H | $OCH_3$ | H | Cl | O | |
| 6.06 | $CH_3$ | H | H | $OC_2H_5$ | H | Cl | O | |
| 6.07 | $CH_3$ | H | H | $SCH_3$ | H | Cl | O | |
| 6.08 | $CH_3$ | H | H | $N(CH_3)_2$ | H | Cl | O | |
| 6.09 | $CH_3$ | H | H | $-OCH_2-CF_3$ | H | Cl | O | |
| 6.10 | $CH_3$ | H | H | $CF_3$ | H | Cl | O | |
| 6.11 | $CH_3$ | $CH_3$ | H | $CH_3$ | H | Cl | O | |
| 6.12 | $CH_3$ | $CH_3$ | H | H | H | Cl | O | |
| 6.13 | $CH_3$ | $CH_3$ | H | Cl | H | Cl | O | |
| 6.14 | $CH_3$ | $CH_3$ | H | $OCH_3$ | H | Cl | O | |
| 6.15 | $C_2H_5$ | H | H | Cl | H | Cl | O | |
| 6.16 | $C_2H_5$ | H | H | Br | H | Cl | O | |
| 6.17 | $C_2H_5$ | H | H | H | H | Cl | O | |
| 6.18 | $C_2H_5$ | H | H | $CH_3$ | H | Cl | O | |
| 6.19 | $C_2H_5$ | H | H | $OCH_3$ | H | Cl | O | |
| 6.20 | $C_2H_5$ | H | H | $N(CH_3)_2$ | H | Cl | O | |
| 6.21 | $C_2H_5$ | H | H | $SCH_3$ | H | Cl | O | |
| 6.22 | $C_2H_5$ | H | H | $OC_2H_5$ | H | Cl | O | |
| 6.23 | $C_2H_5$ | H | H | $-OCH_2-CF_3$ | H | Cl | O | |

| Verb. Nr. | $R^1$ | $R^2$ | $R^3$ | $R^4$ | $R^7$ | $R^8$ | X | Smp. [°C] |
|---|---|---|---|---|---|---|---|---|
| 6.24 | $C_2H_5$ | H | H | $CF_3$ | H | Cl | O | |
| 6.25 | $C_2H_5$ | $CH_3$ | H | $CH_3$ | H | Cl | O | |
| 6.26 | $C_2H_5$ | $CH_3$ | H | H | H | Cl | O | |
| 6.27 | $C_2H_5$ | $CH_3$ | H | Cl | H | Cl | O | |
| 6.28 | $C_2H_5$ | $CH_3$ | H | $OCH_3$ | H | Cl | O | |
| 6.29 | $CH_3$ | H | H | $OC_2H_5$ | H | $COOCH_3$ | O | |
| 6.30 | $CH_3$ | H | H | Cl | H | $COOCH_3$ | O | |
| 6.31 | $CH_3$ | H | H | $OCH_3$ | H | $COOCH_3$ | O | |
| 6.32 | $CH_3$ | H | H | $OC_2H_5$ | H | $OCH_3$ | O | |
| 6.33 | $CH_3$ | H | H | Cl | H | $OCH_3$ | O | |
| 6.33 | $CH_3$ | H | H | $OCH_3$ | H | $OCH_3$ | O | |

Tabelle 7:

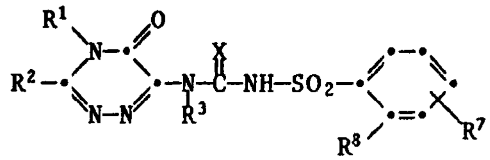

| Verb. Nr. | $R^1$ | $R^2$ | $R^3$ | $R^7$ | $R^8$ | X | Smp.[°C] |
|---|---|---|---|---|---|---|---|
| 7.01 | $CH_3$ | Cl | H | H | $COOCH_3$ | O | |
| 7.02 | $CH_3$ | H | H | H | $COOCH_3$ | O | |
| 7.03 | $CH_3$ | $CH_3$ | H | H | $COOCH_3$ | O | 164-165 (Zers.) |
| 7.04 | $CH_3$ | $OCH_3$ | H | H | $COOCH_3$ | O | 181-182 (Zers.) |
| 7.05 | $CH_3$ | $N(CH_3)_2$ | H | H | $COOCH_3$ | O | |
| 7.06 | $CH_3$ | $SCH_3$ | H | H | $COOCH_3$ | O | 186-187 |
| 7.07 | $CH_3$ | $OC_2H_5$ | H | H | $COOCH_3$ | O | 188 (Zers.) |
| 7.08 | $CH_3$ | $-OCH_2-CF_3$ | H | H | $COOCH_3$ | O | |
| 7.09 | $CH_3$ | $CF_3$ | H | H | $COOCH_3$ | O | |
| 7.10 | $C_2H_5$ | Cl | H | H | $COOCH_3$ | O | |
| 7.11 | $C_2H_5$ | H | H | H | $COOCH_3$ | O | |
| 7.12 | $C_2H_5$ | $CH_3$ | H | H | $COOCH_3$ | O | |
| 7.13 | $C_2H_5$ | $OCH_3$ | H | H | $COOCH_3$ | O | |
| 7.14 | $C_2H_5$ | $N(CH_3)_2$ | H | H | $COOCH_3$ | O | |
| 7.15 | $C_2H_5$ | $SCH_3$ | H | H | $COOCH_3$ | O | |
| 7.16 | $C_2H_5$ | $OC_2H_5$ | H | H | $COOCH_3$ | O | |
| 7.17 | $C_2H_5$ | $-OCH_2-CF_3$ | H | H | $COOCH_3$ | O | |
| 7.18 | $C_2H_5$ | $CF_3$ | H | H | $COOCH_3$ | O | |
| 7.19 | $CH_3$ | $-OCH_2-CH_2OCH_3$ | H | H | $COOCH_3$ | O | 136 (Zers.) |
| 7.20 | $CH_3$ | Cl | H | H | $-SO_2N(CH_3)_2$ | O | |
| 7.21 | $CH_3$ | H | H | H | $-SO_2N(CH_3)_2$ | O | |
| 7.22 | $CH_3$ | $CH_3$ | H | H | $-SO_2N(CH_3)_2$ | O | |
| 7.23 | $CH_3$ | $OCH_3$ | H | H | $-SO_2N(CH_3)_2$ | O | |

| Verb. Nr. | $R^1$ | $R^2$ | $R^3$ | $R^7$ | $R^8$ | X | Smp. [°C] |
|---|---|---|---|---|---|---|---|
| 7.24 | $CH_3$ | $N(CH_3)_2$ | H | H | $-SO_2N(CH_3)_2$ | O | |
| 7.25 | $CH_3$ | $SCH_3$ | H | H | $-SO_2N(CH_3)_2$ | O | |
| 7.26 | $CH_3$ | $OC_2H_5$ | H | H | $-SO_2N(CH_3)_2$ | O | |
| 7.27 | $CH_3$ | $-OCH_2-CF_3$ | H | H | $-SO_2N(CH_3)_2$ | O | |
| 7.28 | $CH_3$ | $CF_3$ | H | H | $-SO_2N(CH_3)_2$ | O | |
| 7.29 | $C_2H_5$ | Cl | H | H | $-SO_2N(CH_3)_2$ | O | |
| 7.30 | $C_2H_5$ | H | H | H | $-SO_2N(CH_3)_2$ | O | |
| 7.31 | $C_2H_5$ | $CH_3$ | H | H | $-SO_2N(CH_3)_2$ | O | |
| 7.32 | $C_2H_5$ | $OCH_3$ | H | H | $-SO_2N(CH_3)_2$ | O | |
| 7.33 | $C_2H_5$ | $N(CH_3)_2$ | H | H | $-SO_2N(CH_3)_2$ | O | |
| 7.34 | $C_2H_5$ | $SCH_3$ | H | H | $-SO_2N(CH_3)_2$ | O | |
| 7.35 | $C_2H_5$ | $OC_2H_5$ | H | H | $-SO_2N(CH_3)_2$ | O | |
| 7.36 | $C_2H_5$ | $-OCH_2-CF_3$ | H | H | $-SO_2N(CH_3)_2$ | O | |
| 7.37 | $C_2H_5$ | $CF_3$ | H | H | $-SO_2N(CH_3)_2$ | O | |
| 7.38 | $CH_3$ | Cl | H | H | $OC_2H_5$ | O | |
| 7.39 | $CH_3$ | H | H | H | $OC_2H_5$ | O | |
| 7.40 | $CH_3$ | $CH_3$ | H | H | $OC_2H_5$ | O | |
| 7.41 | $CH_3$ | $OCH_3$ | H | H | $OC_2H_5$ | O | |
| 7.42 | $CH_3$ | $N(CH_3)_2$ | H | H | $OC_2H_5$ | O | |
| 7.43 | $CH_3$ | $SCH_3$ | H | H | $OC_2H_5$ | O | |
| 7.44 | $CH_3$ | $OC_2H_5$ | H | H | $OC_2H_5$ | O | |
| 7.45 | $CH_3$ | $-OCH_2-CF_3$ | H | H | $OC_2H_5$ | O | |
| 7.46 | $CH_3$ | $CF_3$ | H | H | $OC_2H_5$ | O | |
| 7.47 | $C_2H_5$ | Cl | H | H | $OC_2H_5$ | O | |
| 7.48 | $C_2H_5$ | H | H | H | $OC_2H_5$ | O | |
| 7.49 | $C_2H_5$ | $CH_3$ | H | H | $OC_2H_5$ | O | |
| 7.50 | $C_2H_5$ | $OCH_3$ | H | H | $OC_2H_5$ | O | |
| 7.51 | $C_2H_5$ | $N(CH_3)_2$ | H | H | $OC_2H_5$ | O | |
| 7.52 | $C_2H_5$ | $SCH_3$ | H | H | $OC_2H_5$ | O | |

| Verb. Nr. | $R^1$ | $R^2$ | $R^3$ | $R^7$ | $R^8$ | X | Smp.[°C] |
|---|---|---|---|---|---|---|---|
| 7.53 | $C_2H_5$ | $OC_2H_5$ | H | H | $OC_2H_5$ | O | |
| 7.54 | $C_2H_5$ | $-OCH_2-CF_3$ | H | H | $OC_2H_5$ | O | |
| 7.55 | $C_2H_5$ | $CF_3$ | H | H | $OC_2H_5$ | O | |
| 7.56 | $CH_3$ | Cl | H | H | $OCHF_2$ | O | |
| 7.57 | $CH_3$ | H | H | H | $OCHF_2$ | O | |
| 7.58 | $CH_3$ | $CH_3$ | H | H | $OCHF_2$ | O | 157-158 |
| 7.59 | $CH_3$ | $OCH_3$ | H | H | $OCHF_2$ | O | 159-161 |
| 7.60 | $CH_3$ | $N(CH_3)_2$ | H | H | $OCHF_2$ | O | |
| 7.61 | $CH_3$ | $SCH_3$ | H | H | $OCHF_2$ | O | |
| 7.62 | $CH_3$ | $OC_2H_5$ | H | H | $OCHF_2$ | O | |
| 7.63 | $CH_3$ | $-OCH_2-CF_3$ | H | H | $OCHF_2$ | O | |
| 7.64 | $CH_3$ | $CF_3$ | H | H | $OCHF_2$ | O | |
| 7.65 | $C_2H_5$ | Cl | H | H | $OCHF_2$ | O | |
| 7.66 | $C_2H_5$ | H | H | H | $OCHF_2$ | O | |
| 7.67 | $C_2H_5$ | $CH_3$ | H | H | $OCHF_2$ | O | |
| 7.68 | $C_2H_5$ | $OCH_3$ | H | H | $OCHF_2$ | O | |
| 7.69 | $C_2H_5$ | $N(CH_3)_2$ | H | H | $OCHF_2$ | O | |
| 7.70 | $C_2H_5$ | $SCH_3$ | H | H | $OCHF_2$ | O | |
| 7.71 | $C_2H_5$ | $OC_2H_5$ | H | H | $OCHF_2$ | O | |
| 7.72 | $C_2H_5$ | $-OCH_2-CF_3$ | H | H | $OCHF_2$ | O | |
| 7.73 | $C_2H_5$ | $CF_3$ | H | H | $OCHF_2$ | O | |
| 7.74 | $CH_3$ | Cl | H | H | $CF_3$ | O | |
| 7.75 | $CH_3$ | H | H | H | $CF_3$ | O | |
| 7.76 | $CH_3$ | $CH_3$ | H | H | $CF_3$ | O | |
| 7.77 | $CH_3$ | $OCH_3$ | H | H | $CF_3$ | O | |
| 7.78 | $CH_3$ | $N(CH_3)_2$ | H | H | $CF_3$ | O | |
| 7.79 | $CH_3$ | $SCH_3$ | H | H | $CF_3$ | O | |
| 7.80 | $CH_3$ | $OC_2H_5$ | H | H | $CF_3$ | O | |
| 7.81 | $CH_3$ | $-OCH_2-CF_3$ | H | H | $CF_3$ | O | |
| 7.82 | $CH_3$ | $CF_3$ | H | H | $CF_3$ | O | |

| Verb. Nr. | $R^1$ | $R^2$ | $R^3$ | $R^7$ | $R^8$ | X | Smp. [°C] |
|---|---|---|---|---|---|---|---|
| 7.83 | $C_2H_5$ | Cl | H | H | $CF_3$ | O | |
| 7.84 | $C_2H_5$ | H | H | H | $CF_3$ | O | |
| 7.85 | $C_2H_5$ | $CH_3$ | H | H | $CF_3$ | O | |
| 7.86 | $C_2H_5$ | $OCH_3$ | H | H | $CF_3$ | O | |
| 7.87 | $C_2H_5$ | $N(CH_3)_2$ | H | H | $CF_3$ | O | |
| 7.88 | $C_2H_5$ | $SCH_3$ | H | H | $CF_3$ | O | |
| 7.89 | $C_2H_5$ | $OC_2H_5$ | H | H | $CF_3$ | O | |
| 7.90 | $C_2H_5$ | $-OCH_2-CF_3$ | H | H | $CF_3$ | O | |
| 7.91 | $C_2H_5$ | $CF_3$ | H | H | $CF_3$ | O | |
| 7.92 | $C_3H_7$-i | $OC_2H_5$ | H | H | $COOCH_3$ | O | |
| 7.93 | $C_3H_7$-i | $CH_3$ | H | H | $COOCH_3$ | O | |
| 7.94 | $C_3H_7$-i | $OCH_3$ | H | H | $COOCH_3$ | O | |
| 7.95 | $CH_3$ | $CH_3$ | H | H | $COOCH_3$ | S | |
| 7.96 | $CH_3$ | $CH_3$ | $CH_3$ | H | $COOCH_3$ | O | |
| 7.97 | $CH_3$ | $CH_3$ | $C_2H_5$ | H | $COOCH_3$ | O | |
| 7.98 | $CH_3$ | $CH_3$ | H | 5-$OCH_3$ | $COOCH_3$ | O | |
| 7.99 | $CH_3$ | $CH_3$ | H | 5-F | $COOCH_3$ | O | |
| 7.100 | $CH_3$ | $CH_3$ | H | 6-Cl | $COOCH_3$ | O | |
| 7.101 | $C_3H_7$-i | $OC_2H_5$ | H | H | $OCHF_2$ | O | |
| 7.102 | $C_3H_7$-i | $CH_3$ | H | H | $OCHF_2$ | O | |
| 7.103 | $C_3H_7$-i | $OCH_3$ | H | H | $OCHF_2$ | O | |
| 7.104 | $CH_3$ | $CH_3$ | H | H | $OCHF_2$ | S | |
| 7.105 | $CH_3$ | $CH_3$ | $CH_3$ | H | $OCHF_2$ | O | |
| 7.106 | $CH_3$ | $CH_3$ | $C_2H_5$ | H | $OCHF_2$ | O | |
| 7.107 | $CH_3$ | $CH_3$ | H | 5-$OCH_3$ | $OCHF_2$ | O | |
| 7.108 | $CH_3$ | $CH_3$ | H | 5-F | $OCHF_2$ | O | |
| 7.109 | $CH_3$ | $CH_3$ | H | 6-Cl | $OCHF_2$ | O | |
| 7.110 | $CH_3$ | $OCH_3$ | H | H | $CHF_2$ | O | |
| 7.111 | $CH_3$ | $CH_3$ | H | H | $CHF_2$ | O | |

| Verb. Nr. | $R^1$ | $R^2$ | $R^3$ | $R^7$ | $R^8$ | X | Smp.[°C] |
|---|---|---|---|---|---|---|---|
| 7.112 | $CH_3$ | $OC_2H_5$ | H | H | $CHF_2$ | O | |
| 7.113 | $CH_3$ | $OCH_3$ | H | H | $OCH_3$ | O | |
| 7.114 | $CH_3$ | $CH_3$ | H | H | $OCH_3$ | O | |
| 7.115 | $CH_3$ | $OC_2H_5$ | H | H | $OCH_3$ | O | |
| 7.116 | $CH_3$ | $OCH_3$ | H | H | $-OCH_2-CH_2Cl$ | O | |
| 7.117 | $CH_3$ | $CH_3$ | H | H | $-OCH_2-CH_2Cl$ | O | |
| 7.118 | $CH_3$ | $OC_2H_5$ | H | H | $-OCH_2-CH_2Cl$ | O | |
| 7.119 | $CH_3$ | $OCH_3$ | H | H | $-OCH_2-CH_2OCH_3$ | O | |
| 7.120 | $CH_3$ | $CH_3$ | H | H | $-OCH_2-CH_2OCH_3$ | O | |
| 7.121 | $CH_3$ | $OC_2H_5$ | H | H | $-OCH_2-CH_2OCH_3$ | O | |
| 7.122 | $CH_3$ | $OCH_3$ | H | H | $-OCH_2-CF_3$ | O | |
| 7.123 | $CH_3$ | $CH_3$ | H | H | $-OCH_2-CF_3$ | O | |
| 7.124 | $CH_3$ | $OC_2H_5$ | H | H | $-OCH_2-CF_3$ | O | |
| 7.125 | $CH_3$ | $OCH_3$ | H | H | $-OCCl=CHCl$ | O | |
| 7.126 | $CH_3$ | $CH_3$ | H | H | $-OCCl=CHCl$ | O | |
| 7.127 | $CH_3$ | $OC_2H_5$ | H | H | $-OCCl=CHCl$ | O | |
| 7.128 | $CH_3$ | $OCH_3$ | H | H | $NO_2$ | O | |
| 7.129 | $CH_3$ | $CH_3$ | H | H | $NO_2$ | O | |
| 7.130 | $CH_3$ | $OC_2H_5$ | H | H | $NO_2$ | O | |
| 7.131 | $CH_3$ | $OCH_3$ | H | H | $COOC_2H_5$ | O | |
| 7.132 | $CH_3$ | $CH_3$ | H | H | $COOC_2H_5$ | O | |
| 7.133 | $CH_3$ | $OC_2H_5$ | H | H | $COOC_2H_5$ | O | |
| 7.134 | $CH_3$ | $OCH_3$ | H | H | Cl | O | |
| 7.135 | $CH_3$ | $CH_3$ | H | H | Cl | O | 178-179 |
| 7.136 | $CH_3$ | $OC_2H_5$ | H | H | Cl | O | |
| 7.137 | $CH_3$ | $OCH_3$ | H | H | F | O | |
| 7.138 | $CH_3$ | $CH_3$ | H | H | F | O | |
| 7.139 | $CH_3$ | $OC_2H_5$ | H | H | F | O | |
| 7.140 | $CH_3$ | $OCH_3$ | H | H | Br | O | |

| Verb. Nr. | $R^1$ | $R^2$ | $R^3$ | $R^7$ | $R^8$ | X | Smp. [°C] |
|---|---|---|---|---|---|---|---|
| 7.141 | $CH_3$ | $CH_3$ | H | H | Br | O | |
| 7.142 | $CH_3$ | $OC_2H_5$ | H | H | Br | O | |
| 7.143 | $CH_3$ | $OCH_3$ | H | H | $CH_3$ | O | |
| 7.144 | $CH_3$ | $CH_3$ | H | H | $CH_3$ | O | |
| 7.145 | $CH_3$ | $OC_2H_5$ | H | H | $CH_3$ | O | |
| 7.146 | $CH_3$ | $OCH_3$ | H | H | $SCH_3$ | O | |
| 7.147 | $CH_3$ | $CH_3$ | H | H | $SCH_3$ | O | |
| 7.148 | $CH_3$ | $OC_2H_5$ | H | H | $SCH_3$ | O | |
| 7.149 | $CH_3$ | $OCH_3$ | H | H | $SCHF_2$ | O | |
| 7.150 | $CH_3$ | $CH_3$ | H | H | $SCHF_2$ | O | |
| 7.151 | $CH_3$ | $OC_2H_5$ | H | H | $SCHF_2$ | O | |
| 7.152 | $CH_3$ | $CH_3$ | H | H | $-SCH_2-CH_2Cl$ | O | |
| 7.153 | $CH_3$ | $OCH_3$ | H | H | $-SCH_2-CH_2Cl$ | O | |
| 7.154 | $CH_3$ | $OC_2H_5$ | H | H | $-SCH_2-CH_2Cl$ | O | |
| 7.155 | $CH_3$ | $CH_3$ | H | H | $-SO_2-CH_3$ | O | |
| 7.156 | $CH_3$ | $OC_2H_5$ | H | H | $-SO_2-CH_3$ | O | |
| 7.157 | $CH_3$ | $OCH_3$ | H | H | $-SO_2-CH_3$ | O | |
| 7.158 | $CH_3$ | $CH_3$ | H | H | $-SO_2-C_2H_5$ | O | |
| 7.159 | $CH_3$ | $OC_2H_5$ | H | H | $-SO_2-C_2H_5$ | O | |
| 7.160 | $CH_3$ | $OCH_3$ | H | H | $-SO_2-C_2H_5$ | O | |
| 7.161 | $CH_3$ | $CH_3$ | H | H | $-OCH_2-CH=CH_2$ | O | |
| 7.162 | $CH_3$ | $OC_2H_5$ | H | H | $-OCH_2-CH=CH_2$ | O | |
| 7.163 | $CH_3$ | $OCH_3$ | H | H | $-OCH_2-CH=CH_2$ | O | |
| 7.164 | $CH_3$ | $OCH_3$ | H | H | H | O | |
| 7.165 | $CH_3$ | $CH_3$ | H | H | H | O | |
| 7.166 | $CH_3$ | $OC_2H_5$ | H | H | H | O | |
| 7.167 | $CH_3$ | $OCH_3$ | H | H | $-O-SO_2CH_3$ | O | |
| 7.168 | $CH_3$ | $CH_3$ | H | H | $-O-SO_2CH_3$ | O | |
| 7.169 | $CH_3$ | $OC_2H_5$ | H | H | $-O-SO_2CH_3$ | O | |

**Tabelle 8:**

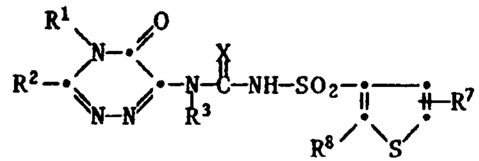

| Verb. Nr. | $R^1$ | $R^2$ | $R^3$ | $R^7$ | $R^8$ | X | Smp.[°C] |
|---|---|---|---|---|---|---|---|
| 8.01 | $CH_3$ | Cl | H | H | $COOCH_3$ | O | |
| 8.02 | $CH_3$ | H | H | H | $COOCH_3$ | O | |
| 8.03 | $CH_3$ | $CH_3$ | H | H | $COOCH_3$ | O | |
| 8.04 | $CH_3$ | $OCH_3$ | H | H | $COOCH_3$ | O | |
| 8.05 | $CH_3$ | $N(CH_3)_2$ | H | H | $COOCH_3$ | O | |
| 8.06 | $CH_3$ | $SCH_3$ | H | H | $COOCH_3$ | O | |
| 8.07 | $CH_3$ | $OC_2H_5$ | H | H | $COOCH_3$ | O | |
| 8.08 | $CH_3$ | $-OCH_2-CF_3$ | H | H | $COOCH_3$ | O | |
| 8.09 | $CH_3$ | $CF_3$ | H | H | $COOCH_3$ | O | |
| 8.10 | $C_2H_5$ | Cl | H | H | $COOCH_3$ | O | |
| 8.11 | $C_2H_5$ | H | H | H | $COOCH_3$ | O | |
| 8.12 | $C_2H_5$ | $CH_3$ | H | H | $COOCH_3$ | O | |
| 8.13 | $C_2H_5$ | $OCH_3$ | H | H | $COOCH_3$ | O | |
| 8.14 | $C_2H_5$ | $N(CH_3)_2$ | H | H | $COOCH_3$ | O | |
| 8.15 | $C_2H_5$ | $SCH_3$ | H | H | $COOCH_3$ | O | |
| 8.16 | $C_2H_5$ | $OC_2H_5$ | H | H | $COOCH_3$ | O | |
| 8.17 | $C_2H_5$ | $-OCH_2-CF_3$ | H | H | $COOCH_3$ | O | |
| 8.18 | $C_2H_5$ | $CF_3$ | H | H | $COOCH_3$ | O | |
| 8.19 | $CH_3$ | $OCH_3$ | H | H | H | O | |
| 8.20 | $CH_3$ | $CH_3$ | H | H | H | O | |
| 8.21 | $CH_3$ | $OC_2H_5$ | H | H | H | O | |
| 8.22 | $CH_3$ | $OCH_3$ | H | H | Cl | O | |
| 8.23 | $CH_3$ | $CH_3$ | H | H | Cl | O | |
| 8.24 | $CH_3$ | $OC_2H_5$ | H | H | Cl | O | |

Tabelle 9:

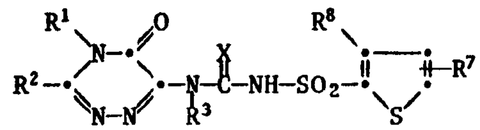

| Verb. Nr. | $R^1$ | $R^2$ | $R^3$ | $R^7$ | $R^8$ | X | Smp. [°C] |
|---|---|---|---|---|---|---|---|
| 9.01 | $CH_3$ | Cl | H | H | $COOCH_3$ | O | |
| 9.02 | $CH_3$ | H | H | H | $COOCH_3$ | O | |
| 9.03 | $CH_3$ | $CH_3$ | H | H | $COOCH_3$ | O | |
| 9.04 | $CH_3$ | $OCH_3$ | H | H | $COOCH_3$ | O | |
| 9.05 | $CH_3$ | $N(CH_3)_2$ | H | H | $COOCH_3$ | O | |
| 9.06 | $CH_3$ | $SCH_3$ | H | H | $COOCH_3$ | O | |
| 9.07 | $CH_3$ | $OC_2H_5$ | H | H | $COOCH_3$ | O | |
| 9.08 | $CH_3$ | $-OCH_2-CF_3$ | H | H | $COOCH_3$ | O | |
| 9.09 | $CH_3$ | $CF_3$ | H | H | $COOCH_3$ | O | |
| 9.10 | $C_2H_5$ | Cl | H | H | $COOCH_3$ | O | |
| 9.11 | $C_2H_5$ | H | H | H | $COOCH_3$ | O | |
| 9.12 | $C_2H_5$ | $CH_3$ | H | H | $COOCH_3$ | O | |
| 9.13 | $C_2H_5$ | $OCH_3$ | H | H | $COOCH_3$ | O | |
| 9.14 | $C_2H_5$ | $N(CH_3)_2$ | H | H | $COOCH_3$ | O | |
| 9.15 | $C_2H_5$ | $SCH_3$ | H | H | $COOCH_3$ | O | |
| 9.16 | $C_2H_5$ | $OC_2H_5$ | H | H | $COOCH_3$ | O | |
| 9.17 | $C_2H_5$ | $-OCH_2-CF_3$ | H | H | $COOCH_3$ | O | |
| 9.18 | $C_2H_5$ | $CF_3$ | H | H | $COOCH_3$ | O | |
| 9.19 | $CH_3$ | $OCH_3$ | H | H | H | O | |
| 9.20 | $CH_3$ | $CH_3$ | H | H | H | O | |
| 9.21 | $CH_3$ | $OC_2H_5$ | H | H | H | O | |
| 9.22 | $CH_3$ | $OCH_3$ | H | H | Cl | O | |
| 9.23 | $CH_3$ | $CH_3$ | H | H | Cl | O | |
| 9.24 | $CH_3$ | $OC_2H_5$ | H | H | Cl | O | |

**Tabelle 10:**

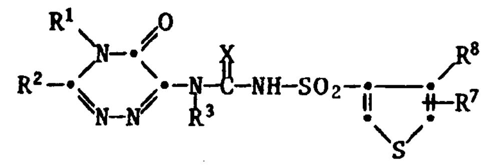

| Verb. Nr. | $R^1$ | $R^2$ | $R^3$ | $R^7$ | $R^8$ | X | Smp.[°C] |
|---|---|---|---|---|---|---|---|
| 10.01 | $CH_3$ | Cl | H | H | $COOCH_3$ | O | |
| 10.02 | $CH_3$ | H | H | H | $COOCH_3$ | O | |
| 10.03 | $CH_3$ | $CH_3$ | H | H | $COOCH_3$ | O | |
| 10.04 | $CH_3$ | $OCH_3$ | H | H | $COOCH_3$ | O | |
| 10.05 | $CH_3$ | $N(CH_3)_2$ | H | H | $COOCH_3$ | O | |
| 10.06 | $CH_3$ | $SCH_3$ | H | H | $COOCH_3$ | O | |
| 10.07 | $CH_3$ | $OC_2H_5$ | H | H | $COOCH_3$ | O | |
| 10.08 | $CH_3$ | $-OCH_2-CF_3$ | H | H | $COOCH_3$ | O | |
| 10.09 | $CH_3$ | $CF_3$ | H | H | $COOCH_3$ | O | |
| 10.10 | $C_2H_5$ | Cl | H | H | $COOCH_3$ | O | |
| 10.11 | $C_2H_5$ | H | H | H | $COOCH_3$ | O | |
| 10.12 | $C_2H_5$ | $CH_3$ | H | H | $COOCH_3$ | O | |
| 10.13 | $C_2H_5$ | $OCH_3$ | H | H | $COOCH_3$ | O | |
| 10.14 | $C_2H_5$ | $N(CH_3)_2$ | H | H | $COOCH_3$ | O | |
| 10.15 | $C_2H_5$ | $SCH_3$ | H | H | $COOCH_3$ | O | |
| 10.16 | $C_2H_5$ | $OC_2H_5$ | H | H | $COOCH_3$ | O | |
| 10.17 | $C_2H_5$ | $-OCH_2-CF_3$ | H | H | $COOCH_3$ | O | |
| 10.18 | $C_2H_5$ | $CF_3$ | H | H | $COOCH_3$ | O | |
| 10.19 | $CH_3$ | $OCH_3$ | H | H | H | O | |
| 10.20 | $CH_3$ | $CH_3$ | H | H | H | O | |
| 10.21 | $CH_3$ | $OC_2H_5$ | H | H | H | O | |
| 10.22 | $CH_3$ | $OCH_3$ | H | H | Cl | O | |
| 10.23 | $CH_3$ | $CH_3$ | H | H | Cl | O | |
| 10.24 | $CH_3$ | $OC_2H_5$ | H | H | Cl | O | |

Tabelle 11:

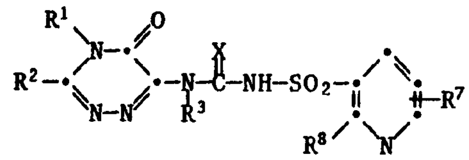

| Verb. Nr. | $R^1$ | $R^2$ | $R^3$ | $R^7$ | $R^8$ | X | Smp.[°C] |
|---|---|---|---|---|---|---|---|
| 11.01 | $CH_3$ | Cl | H | H | Cl | O | |
| 11.02 | $CH_3$ | H | H | H | Cl | O | |
| 11.03 | $CH_3$ | $CH_3$ | H | H | Cl | O | |
| 11.04 | $CH_3$ | $OCH_3$ | H | H | Cl | O | |
| 11.05 | $CH_3$ | $N(CH_3)_2$ | H | H | Cl | O | |
| 11.06 | $CH_3$ | $SCH_3$ | H | H | Cl | O | |
| 11.07 | $CH_3$ | $OC_2H_5$ | H | H | Cl | O | |
| 11.08 | $CH_3$ | $-OCH_2-CF_3$ | H | H | Cl | O | |
| 11.09 | $CH_3$ | $CF_3$ | H | H | Cl | O | |
| 11.10 | $C_2H_5$ | Cl | H | H | Cl | O | |
| 11.11 | $C_2H_5$ | H | H | H | Cl | O | |
| 11.12 | $C_2H_5$ | $CH_3$ | H | H | Cl | O | |
| 11.13 | $C_2H_5$ | $OCH_3$ | H | H | Cl | O | |
| 11.14 | $C_2H_5$ | $N(CH_3)_2$ | H | H | Cl | O | |
| 11.15 | $C_2H_5$ | $SCH_3$ | H | H | Cl | O | |
| 11.16 | $C_2H_5$ | $OC_2H_5$ | H | H | Cl | O | |
| 11.17 | $C_2H_5$ | $-OCH_2-CF_3$ | H | H | Cl | O | |
| 11.18 | $C_2H_5$ | $CF_3$ | H | H | Cl | O | |
| 11.19 | $CH_3$ | $OCH_3$ | H | H | $COOCH_3$ | O | |
| 11.20 | $CH_3$ | $CH_3$ | H | H | $COOCH_3$ | O | |
| 11.21 | $CH_3$ | $OC_2H_5$ | H | H | $COOCH_3$ | O | |
| 11.22 | $CH_3$ | $OCH_3$ | H | H | $OCH_3$ | O | |
| 11.23 | $CH_3$ | $CH_3$ | H | H | $OCH_3$ | O | |
| 11.24 | $CH_3$ | $OC_2H_5$ | H | H | $OCH_3$ | O | |

**Tabelle 12:**

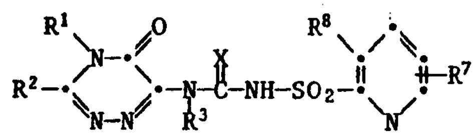

| Verb. Nr. | $R^1$ | $R^2$ | $R^3$ | $R^7$ | $R^8$ | X | Smp.[°C] |
|---|---|---|---|---|---|---|---|
| 12.01 | $CH_3$ | Cl | H | H | Cl | O | |
| 12.02 | $CH_3$ | H | H | H | Cl | O | |
| 12.03 | $CH_3$ | $CH_3$ | H | H | Cl | O | |
| 12.04 | $CH_3$ | $OCH_3$ | H | H | Cl | O | |
| 12.05 | $CH_3$ | $N(CH_3)_2$ | H | H | Cl | O | |
| 12.06 | $CH_3$ | $SCH_3$ | H | H | Cl | O | |
| 12.07 | $CH_3$ | $OC_2H_5$ | H | H | Cl | O | |
| 12.08 | $CH_3$ | $-OCH_2-CF_3$ | H | H | Cl | O | |
| 12.09 | $CH_3$ | $CF_3$ | H | H | Cl | O | |
| 12.10 | $C_2H_5$ | Cl | H | H | Cl | O | |
| 12.11 | $C_2H_5$ | H | H | H | Cl | O | |
| 12.12 | $C_2H_5$ | $CH_3$ | H | H | Cl | O | |
| 12.13 | $C_2H_5$ | $OCH_3$ | H | H | Cl | O | |
| 12.14 | $C_2H_5$ | $N(CH_3)_2$ | H | H | Cl | O | |
| 12.15 | $C_2H_5$ | $SCH_3$ | H | H | Cl | O | |
| 12.16 | $C_2H_5$ | $OC_2H_5$ | H | H | Cl | O | |
| 12.17 | $C_2H_5$ | $-OCH_2-CF_3$ | H | H | Cl | O | |
| 12.18 | $C_2H_5$ | $CF_3$ | H | H | Cl | O | |
| 12.19 | $CH_3$ | $OCH_3$ | H | H | $COOCH_3$ | O | |
| 12.20 | $CH_3$ | $CH_3$ | H | H | $COOCH_3$ | O | |
| 12.21 | $CH_3$ | $OC_2H_5$ | H | H | $COOCH_3$ | O | |
| 12.22 | $CH_3$ | $OCH_3$ | H | H | $OCH_3$ | O | |
| 12.23 | $CH_3$ | $CH_3$ | H | H | $OCH_3$ | O | |
| 12.24 | $CH_3$ | $OC_2H_5$ | H | H | $OCH_3$ | O | |

Tabelle 13:

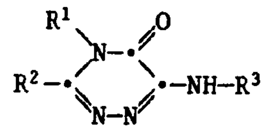

| Verb. Nr. | $R^1$ | $R^2$ | $R^3$ | Smp.[°C] |
|---|---|---|---|---|
| 13.01 | $CH_3$ | Cl | H | |
| 13.02 | $CH_3$ | H | H | |
| 13.03 | $CH_3$ | $OCH_3$ | H | 198-199 |
| 13.05 | $CH_3$ | $N(CH_3)_2$ | H | |
| 13.07 | $CH_3$ | $OC_2H_5$ | H | 167-168 |
| 13.08 | $CH_3$ | $-OCH_2-CF_3$ | H | |
| 13.09 | $CH_3$ | $CF_3$ | H | |
| 13.06 | $CH_3$ | $SCH_3$ | H | 234 |
| 13.12 | $CH_3$ | $CH_3$ | H | 205 |
| 13.10 | $C_2H_5$ | Cl | H | |
| 13.11 | $C_2H_5$ | H | H | |
| 13.12 | $C_2H_5$ | $CH_3$ | H | |
| 13.13 | $C_2H_5$ | $OCH_3$ | H | |
| 13.14 | $C_2H_5$ | $N(CH_3)_2$ | H | |
| 13.15 | $C_2H_5$ | $SCH_3$ | H | |
| 13.16 | $C_2H_5$ | $OC_2H_5$ | H | |
| 13.17 | $C_2H_5$ | $-OCH_2-CF_3$ | H | |
| 13.18 | $C_2H_5$ | $CF_3$ | H | |
| 13.19 | $C_2H_7$-i | $OC_2H_5$ | H | |
| 13.20 | $C_2H_7$-i | $CH_3$ | H | |
| 13.21 | $C_2H_7$-i | $OCH_3$ | H | |
| 13.22 | $CH_3$ | $CH_3$ | $CH_3$ | |
| 13.23 | $CH_3$ | $CH_3$ | $C_2H_5$ | |
| 13.24 | $CH_3$ | $-OCH_2-CH_2OCH_3$ | H | 141-143 |
| 13.25 | $CH_3$ | $SOCH_3$ | H | 189 (Zers.) |
| 13.26 | $CH_3$ | $SO_2CH_3$ | H | 160 (Zers.) |

Tabelle 14:

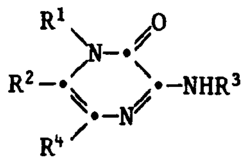

| Verb. Nr. | $R^1$ | $R^2$ | $R^3$ | $R^4$ | Smp.[°C] |
|---|---|---|---|---|---|
| 14.01 | $CH_3$ | H | H | Br | 251-253 |
| 14.02 | $CH_3$ | H | H | $CH_3$ | |
| 14.03 | $CH_3$ | H | H | $OCH_3$ | 161-162 |
| 14.04 | $CH_3$ | H | H | $N(CH_3)_2$ | |
| 14.05 | $CH_3$ | H | H | $SCH_3$ | |
| 14.06 | $CH_3$ | H | H | $OC_2H_5$ | |
| 14.07 | $CH_3$ | H | H | $-OCH_2-CF_3$ | |
| 14.08 | $CH_3$ | H | H | $CF_3$ | |
| 14.09 | $CH_3$ | H | H | H | 161-162 |
| 14.10 | $CH_3$ | H | H | Cl | 265-266 |
| 14.11 | $CH_3$ | $CH_3$ | H | $CH_3$ | |
| 14.12 | $CH_3$ | $CH_3$ | H | H | 217-219 |
| 14.13 | $CH_3$ | $CH_3$ | H | Cl | |
| 14.14 | $CH_3$ | $CH_3$ | H | $OCH_3$ | |
| 14.15 | $C_2H_5$ | H | H | Cl | |
| 14.16 | $C_2H_5$ | H | H | Br | 227-230 |
| 14.17 | $C_2H_5$ | H | H | H | |
| 14.18 | $C_2H_5$ | H | H | $CH_3$ | |
| 14.19 | $C_2H_5$ | H | H | $OCH_3$ | |
| 14.20 | $C_2H_5$ | H | H | $N(CH_3)_2$ | |
| 14.21 | $C_2H_5$ | H | H | $SCH_3$ | |
| 14.22 | $C_2H_5$ | H | H | $OC_2H_5$ | |
| 14.23 | $C_2H_5$ | H | H | $-OCH_2-CF_3$ | |
| 14.24 | $C_2H_5$ | H | H | $CF_3$ | |
| 14.25 | $C_2H_5$ | $CH_3$ | H | $CH_3$ | |
| 14.26 | $C_2H_5$ | $CH_3$ | H | H | |
| 14.27 | $C_2H_5$ | $CH_3$ | H | Cl | |
| 14.28 | $C_2H_5$ | $CH_3$ | H | $OCH_3$ | |
| 14.29 | $C_3H_7-i$ | H | H | Cl | |
| 14.30 | $C_3H_7-i$ | H | H | $CH_3$ | |
| 14.31 | $C_3H_7-i$ | H | H | $OCH_3$ | |
| 14.32 | $CH_3$ | H | $CH_3$ | Cl | 155-156 |
| 14.33 | $CH_3$ | H | $C_2H_5$ | Cl | |
| 14.34 | $CH_3$ | $OCH_3$ | H | H | |
| 14.35 | $C_2H_5$ | $OCH_3$ | H | H | |
| 14.36 | $CH_3$ | $OC_2H_5$ | H | H | |
| 14.37 | $CH_3$ | $SCH_3$ | H | H | |
| 14.38 | $CH_3$ | $N(CH_3)_2$ | H | H | |
| 14.39 | $CH_3$ | $OCH_3$ | H | Br | |
| 14.40 | $CH_3$ | $OCH_3$ | H | Cl | |
| 14.41 | $CH_3$ | Br | H | Cl | |
| 14.42 | $CH_3$ | H | H | $-CH_2-OCH_3$ | |
| 14.43 | $CH_3$ | Cl | H | Cl | |

Formulierungsbeispiele:

Beispiel F 1: Formulierungsbeispiele für Wirkstoffe der Formel I (% = Gewichtsprozent)

| a) Spritzpulver | a) | b) | c) |
|---|---|---|---|
| Wirkstoff Nr. 7.04 | 20 % | 50 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther /7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff Nr. 1.01 | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff Nr. 7.07 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff Nr. 1.05 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff Nr. 1.03 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff Nr. 7.07 | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff Nr. 1.29 | 5 % |
| Isopropylamin | 1 % |
| Octylphenolpolyäthylenglykoläther (78 Mol AeO) | 3 % |
| Wasser | 91 % |

Biologische Beispiele:

Beispiel B 1: Herbizidwirkung vor dem Auflaufen der Pflanzen

Kunststofftöpfe werden mit expandiertem Vermiculit (Dichte: 0,135 g/cm$^3$, Wasserabsorptionsvermögen: 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während einer Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5.Tag wird dem Giesswasser 0,5 % eines handelsüblichen Flüssigdüngers (®Greenzit, ex Ciba-Geigy) zugesetzt. 12 Tage nach der Aussaat wird der Versuch ausgewertet und die Wirkung auf die Versuchspflanzen nach dem folgenden Massstab bewertet:

1 : Pflanze nicht gekeimt oder total abgestorben
2-3: sehr starke Wirkung

4-6: mittlere Wirkung
7-8: schwache Wirkung
9: keine Wirkung (wie unbehandelte Kontrolle).

pre-emergente Wirkung:

Konzentration der Wirkstoffemulsion: 70,8 ppm

| Testpflanze Wirkstoff Nr | Nasturtium | Stellaria | Agrostis | Digitaria |
|---|---|---|---|---|
| 1.01 | 2 | 2 | 2 | 2 |
| 1.03 | 2 | 2 | 2 | 2 |
| 1.05 | 2 | 3 | 2 | 3 |
| 1.11 | 2 | 2 | 2 | 2 |
| 1.12 | 2 | 3 | 2 | 3 |
| 1.29 | 2 | 2 | 2 | 2 |
| 1.57 | 2 | 2 | 2 | 2 |
| 1.61 | 2 | 3 | 2 | 3 |
| 1.85 | 2 | 2 | 2 | 2 |
| 1.89 | 2 | 3 | 2 | 3 |
| 1.215 | 2 | 2 | 2 | 2 |
| 1.218 | 2 | 3 | 2 | 3 |
| 5.01 | 3 | 3 | 3 | 3 |
| 7.03 | 3 | 6 | 3 | 7 |
| 7.04 | 1 | 1 | 1 | 1 |
| 7.06 | 2 | 2 | 2 | 2 |
| 7.07 | 2 | 2 | 2 | 2 |
| 7.19 | 2 | 2 | 2 | 2 |
| 7.58 | 3 | 4 | 3 | 5 |
| 7.59 | 2 | 2 | 2 | 2 |
| 7.135 | 2 | 2 | 6 | 7 |

Beispiel B 2: Wuchshemmung bei tropischen Bodenbedecker-Leguminosen (cover crops).

Die Versuchspflanzen (Centrosema plumieri und Centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 600 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt werden.

Beispiel B 3: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Dünger zugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten im Haupttrieb.

Beispiel B 4: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel B 5: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I bewirken eine Reduzierung des Neuzuwachses von um 10-30 % im Vergleich zur unbehandelten Kontrolle.

## Patentansprüche

1. Aminopyrazinone und Aminotriazinone der Formel I

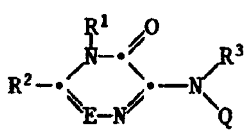 (I),

worin

E Sticksoff oder $=CR^4-$,

$R^1$ $C_1$-$C_4$-Alkyl,

$R^2$ Wasserstoff, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, $C_1$-$C_3$-Alkylsulfinyl, $C_1$-$C_2$-Alkoxyäthoxy, $C_1$-$C_3$-Alkylsulfonyl, Halogen oder $-NR^5R^6$,

$R^3$ Wasserstoff oder $C_1$-$C_3$-Alkyl und

Q eine Gruppe

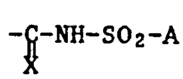

bedeuten,

wobei

$R^4$ für Wasserstoff, $C_1$-$C_3$-Alkyl, $C_1$-$C_3$-Halogenalkyl, $C_1$-$C_3$-Alkoxy, Cyclopropyl, $C_1$-$C_3$-Halogenalkoxy, $C_1$-

$C_3$-Alkylthio, $C_2$-$C_4$-Alkoxyalkyl, $C_3$-$C_5$-Dialkoxymethyl, Halogen oder -$NR^5R^6$,

$R^5$ und $R^6$ unabhängig voneinander für Wasserstoff oder $C_1$-$C_3$-Alkyl,

X für Sauerstoff oder Schwefel und

A für eine Gruppe

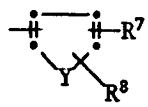

stehen, worin

Y Saueistoff, Schwefel, -CH=CH-, -$NR^9$- oder -$CR^{10}$=N-,

$R^7$ Wasserstoff, Halogen, $C_1$-$C_3$-Alkyl, $C_1$-$C_3$-Alkoxy, Nitro oder Trifluormethyl,

$R^8$ Wasserstoff, Halogen, $C_1$-$C_3$-Alkyl, $C_1$-$C_3$-Alkoxy, Nitro, -C≡CH, oder eine der Gruppen

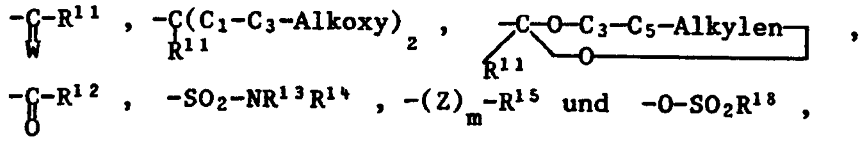

$R^9$ und $R^{10}$ unabhängig voneinander Wasserstoff, $C_1$-$C_3$-Alkyl oder $C_2$-$C_4$-Alkenyl,

$R^{11}$ Wasserstoff, $C_1$-$C_3$-Alkyl, $C_1$-$C_3$-Halogenalkyl, $C_3$-$C_6$-Cycloalkyl oder $C_2$-$C_4$-Alkoxyalkyl,

$R^{12}$ $C_1$-$C_6$-Alkoxy, $C_3$-$C_6$-Alkenyloxy, $C_3$-$C_6$-Alkinyloxy, $C_2$-$C_6$-Halogenalkoxy, $C_1$-$C_4$-Cyanoalkoxy, $C_1$-$C_6$-Alkylthio, $C_3$-$C_6$-Alkenylthio, $C_3$-$C_6$-Alkinylthio, $C_5$-$C_6$-Cycloalkoxy, $C_2$-$C_6$-Alkoxyalkoxy oder -$NR^{16}R^{17}$,

$R^{13}$ Wasserstoff, $C_1$-$C_3$-Alkyl oder $C_3$-$C_4$-Alkenyl,

$R^{14}$ Wasserstoff, $C_1$-$C_3$-Alkyl, $C_1$-$C_3$-Cyanoalkyl oder $C_1$-$C_3$-Alkoxy,

$R^{15}$ $C_3$-$C_6$-Alkinyl, $C_2$-$C_6$-Alkenyl, $C_1$-$C_6$-Alkyl, $C_1$-$C_4$-Halogenalkyl, $C_2$-$C_4$-Halogenalkenyl, $C_1$-$C_4$-Alkyl substituiert durch Cyano, Methoxy, Aethoxy, Nitro, $C_1$-$C_4$-Alkoxycarbonyl, Methylthio, Aethylthio, Methylsulfonyl oder Aethylsulfonyl; oder $C_3$-$C_4$-Alkenyl substituiert durch Nitro, Cyano, Methoxy oder Aethoxy,

$R^{16}$ Wasserstoff, $C_1$-$C_3$-Alkyl oder $C_3$-$C_4$-Alkenyl,

$R^{17}$ Wasserstoff, $C_1$-$C_3$-Alkyl, $C_1$-$C_3$-Cyanoalkyl oder $C_1$-$C_3$-Alkoxy,

$R^{18}$ $C_1$-$C_3$-Alkyl oder $C_1$-$C_3$-Halogenalkyl,

W Sauerstoff oder Schwefel,

Z Sauerstoff, Schwefel, -SO- oder -$SO_2$-, und

m die Zahl null oder eins bedeuten,

sowie die Salze dieser Verbindungen.

2. Verbindungen gemäss Anspruch 1, dadurch gekennzeichnet, dass X Sauerstoff bedeutet.

3. Verbindungen gemäss Anspruch 1, dadurch gekennzeichnet, dass A für eine Gruppe

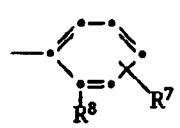

steht.

4. Verbindungen gemäss Anspruch 3, dadurch gekennzeichnet, dass $R^7$ Wasserstoff und $R^8$ Methoxycarbonyl, Aethoxycarbonyl, Dimethylaminosulfonyl, Propoxy, Aethoxy, Difluormethoxy, Trifluormethyl, Chloräthoxy, Methoxyäthoxy, 2,2,2-Trifluoräthoxy, 1,2-Dichlominyloxy, Nitro, Fluor, Clor, Brom, Methyl, Methylthio, Difluormethylthio, Chloräthylthio, -O-$SO_2CH_3$, Allyloxy oder Methoxy bedeuten.

5. Verbindungen gemäss Anspruch 1, dadurch gekennzeichnet, dass $R_3$ Wasserstoff bedeutet.

6. Verbindungen gemäss Anspruch 1, dadurch gekennzeichnet, dass $R^1$ Methyl oder Aethyl, $R^2$ Wasserstoff, Methoxy oder Methyl und E die Gruppe =$CR^4$-bedeuten.

7. Verbindungen gemäss Anspruch 1, dadurch gekennzeichnet, dass $R^1$ Methyl oder Aethyl, $R^2$ Methoxy, Aethoxy, Methylthio, Dimethylamino, Methyl, Trifluormethyl, 2,2,2-Trifluoräthoxy oder Aethyl und E Stickstoff bedeuten.

8. Verbindungen gemäss Anspruch 6 dadurch gekennzeichnet, dass $R^4$ Chlor, Brom,Methoxy, Aethoxy, Methyl, Aethyl, Methylthio, Direthylamino, Trifluormethyl oder 2,2,2-Trifluoräthoxy bedeutet.

9. Verbindungen gemäss Anspruch 1, dadurch gekennzeichnet, dass $R^1$ Methyl oder Aethyl, $R^2$ Wasserstoff, Methoxy oder Methyl, $R^3$ Wasserstoff, X Sauerstoff, A die Gruppe

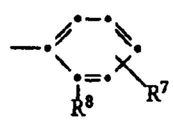 ,

$R^7$ Wasserstoff, $R^8$ Methoxycarbonyl, Aethoxycarbonyl, Dimethylarinosulfonyl, Methoxy, Aethoxy, Difluormethoxy, Trifluormethyl, Chloräthoxy, Methoxyäthoxy, 2,2,2-Trifluoräthoxy, 1,2-Dichlorvinyloxy, Nitro, Fluor, Chlor, Brom, Methyl, Methylthio, Difluormethylthio, Chloräthylthio, $-O-SO_2CH_3$ oder Allyloxy, E die Gruppe $=CR^4-$ und $R^4$ Chlor, Brom, Methoxy, Aethoxy, Methyl, Aethyl, Methylthio, Dimethylamino, Trifluormethyl oder 2,2,2-Trifluoräthoxy bedeuten.

10. Verbindungen gemäss Anspruch 1, dadurch gekennzeichnet, dass $R^1$ Methyl oder Aethyl, $R^2$ Methoxy, Aethoxy, Methylthio, Dimethylamino, Methyl, Trifluormethyl, 2,2,2-Trifluoräthoxy oder Aethyl, $R^3$ Wasserstoff, E Stickstoff, X Sauerstoff, A die Gruppe

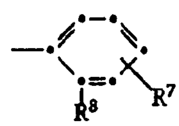 ,

$R^7$ Wasserstoff und $R^8$ Methoxycarbonyl, Aethoxycarbonyl, Direthylaminosulfonyl, Methoxy, Aethoxy, Difluormethoxy, Trifluormethyl, Chloräthoxy, Methoxyäthoxy, 2,2,2-Trifluoräthoxy, 1,2-Dichlorvinyloxy, Nitro, $-O-SO_2CH_3$, Fluor, Chlor, Brom, Methyl, Methylthio, Difluormethylthio, Chloräthylthio oder Allyloxy bedeuten.

11. 6-[3-(2-Methoxycarbonylphenylsulfonyl)-ureido]-3-methoxy-4-methyl-1,2,4-triazin-5-on gemäss Anspruch 1.

12. 6-[3-(2-Methoxycarbonylphenylsulfonyl)-ureido]-5-chlor-1-methyl-pyrazin-2-on gemäss Anspruch 1.

13. Verfahren zur Herstellung von Verbindungen der Formel I, dadurch gekennzeichnet, dass ran ein Aminopyrazinon oder Aminotriazinon der Formel II

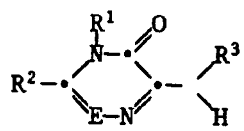 (II)

worin $R^1$, $R^2$, $R^3$ und E die unter Formel I gegebene Bedeutung haben, mit einem Sulfonylisocyanat der Formel III

$$X = C = N\text{-}SO_2\text{-}A \quad \text{(III)}$$

worin A und X die unter Formel I gegebenen Bedeutungen haben, umsetzt und gegebenenfalls in ein Salz überführt.

14. Verfahren zur Herstellung der Verbindungen der Formel I, dadurch gekennzeichnet, dass man ein Aminopyrazinon oder Aminotriazinon der Formel II gemäss Anspruch 13 in Gegenwart einer Base mit einem Sulfonylcarbamat der Formel IV

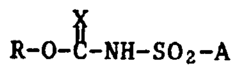 (IV)

worin A und X die unter Formel I gegebene Bedeutung haben und R für Phenyl, Alkyl oder substituiertes Phenyl steht, umsetzt und gegebenenfalls in ein Salz überführt.

15. Verfahren zur Herstellung der Verbindungen der Formel I, dadurch gekennzeichnet, dass man ein Carbarat der Formel V

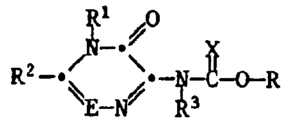

(V)

worin $R^1$, $R^2$, $R^3$, E und X die unter Formel I gegebene Bedeutung haben und R für Phenyl, Alkyl oder substituiertes Phenyl steht, in Gegenwart einer Base mit einem Sulfonamid der Formel VI

$H_2N$-$SO_2$-A (VI)

worin A die unter Formel I gegebene Bedeutung hat, umsetzt und gegebenenfalls in ein Salz überführt.

16. Verfahren zur Herstellung von Salzen der Formel I gemäss einem der Ansprüche 12 bis 15, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

17. Ein herbizides und den Pflanzenwuchs hemmendes Mittel, dadurch gekennzeichnet, dass es neben Träger- und/oder anderen Zuschlagstoffen als Wirkstoff mindestens einen substituierten Sulfonylharnstoff der Formel I, Anspruch 1, enthält.

18. Verfahren zur Bekämpfung unerwünschten Pflanzenwachstums, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

19. Verfahren zur Hemmung des Pflanzenwachstums, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

20. Verfahren zur Beeinflussung des Pflanzenwachstums zum Zweck der Ertragssteigerung, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

21. Verfahren gemäss Anspruch 18, zur selektiven pre- oder postemergenten Bekämpfung von Unkräutern in Nutzpflanzenkulturen.

22. Verfahren gemäss Anspruch 19, zur Unterdrückung des Pflanzenwachstums über das 2-Blattstadium hinaus, dadurch gekennzeichnet, dass die Wirkstoffe preemergent angewendet werden.

23. Verbindungen der Formel II

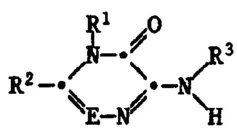

(II)

worin

E Stickstoff oder $=CR^4$-,

$R^1$ $C_1$-$C_4$-Alkyl,

$R_2$ Wasserstoff, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, $C_1$-$C_3$-Alkylsulfinyl, $C_1$-$C_2$-Alkoxyäthoxy, $C_1$-$C_3$-Alkylsulfonyl, Halogen oder $-NR^5R^6$,

$R^3$ Wasserstoff oder $C_1$-$C_3$-Alkyl,

$R^4$ für Wasserstoff, $C_1$-$C_3$-Alkyl, $C_1$-$C_3$-Halogenalkyl, $C_1$-$C_3$-Alkoxy, Cyclopropyl, $C_1$-$C_3$-Halogenalkoxy, $C_1$-$C_3$-Alkylthio, $C_2$-$C_4$-Alkoxyalkyl, $C_3$-$C_5$-Dialkoxymethyl, Halogen oder $-NR^5R^6$ und

$R^5$ und $R^6$ unabhängig voneinander Wasserstoff oder $C_1$-$C_3$-Alkyl bedeuten,

mit der Massgabe, dass $R^3$ für $C_1$-$C_3$-Alkyl steht, wenn

a) gleichzeitig E Stickstoff, $R^1$ Methyl und $R^2$ Methyl oder Methylthio; oder

b) gleichzeitig $R^1$ Methyl, $R^2$ Wasserstoff und E die Gruppe =CCl- oder =CH- bedeuten.

## Claims

1. An aminopyrazinone or aminotriazinone of the formula I

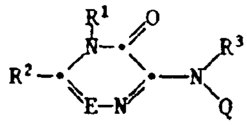

(I)

in which

E is nitrogen or $=CR^4-$,

$R^1$ is $C_1$-$C_4$alkyl,

$R^2$ is hydrogen, $C_1$-$C_3$alkyl, $C_1$-$C_3$halogenoalkyl, $C_1$-$C_3$alkoxy, $C_1$-$C_3$halogenoalkoxy, $C_1$-$C_3$alkylthio, $C_1$-$C_3$alkylsulfinyl, $C_1$-$C_2$alkoxyethoxy, $C_1$-$C_3$alkylsulfonyl, halogen or $-NR^5R^6$,

$R^3$ is hydrogen or $C_1$-$C_3$alkyl and

Q is a group

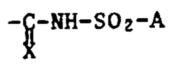

in which

$R^4$ is hydrogen, $C_1$-$C_3$alkyl, $C_1$-$C_3$halogenoalkyl, $C_1$-$C_3$alkoxy, cyclopropyl, $C_1$-$C_3$halogenoalkoxy, $C_1$-$C_3$alkylthio, $C_2$-$C_4$alkoxyalkyl, $C_3$-$C_5$dialkoxymethyl, halogen or $-NR^5R^6$,

$R^5$ and $R^6$ independently of one another are hydrogen or $C_1$-$C_3$alkyl,

X is oxygen or sulfur and

A is a group

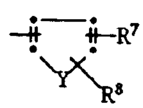

in which

Y is oxygen, sulfur, -CH=CH-, $-NR^9-$ or $-CR^{10}=N-$,

$R^7$ is hydrogen, halogen, $C_1$-$C_3$alkyl, $C_1$-$C_3$alkoxy, nitro or trifluoromethyl,

$R^8$ is hydrogen, halogen, $C_1$-$C_3$alkyl, $C_1$-$C_3$alkoxy, nitro, $-C\equiv CH$, or one of the groups

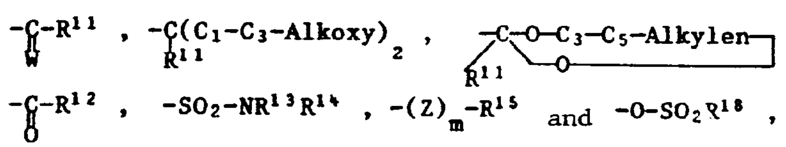

, , , , , , and ,

$R^9$ and $R^{10}$ independently of one another are hydrogen, $C_1$-$C_3$alkyl or $C_2$-$C_4$alkenyl,

$R^{11}$ is hydrogen, $C_1$-$C_3$alkyl, $C_1$-$C_3$halogenoalkyl, $C_3$-$C_6$cycloalkyl or $C_2$-$C_4$alkoxyalkyl,

$R^{12}$ is $C_1$-$C_6$alkoxy, $C_3$-$C_6$alkenyloxy, $C_3$-$C_6$alkinyloxy, $C_2$-$C_6$halogenoalkoxy, $C_1$-$C_4$cyanoalkoxy, $C_1$-$C_6$alkylthio, $C_3$-$C_6$alkenylthio, $C_3$-$C_6$alkinylthio, $C_5$-$C_6$cycloalkoxy, $C_2$-$C_6$alkoxyalkoxy or $-NR^{16}R^{17}$,

$R^{13}$ is hydrogen, $C_1$-$C_3$alkyl or $C_3$-$C_4$alkenyl,

$R^{14}$ is hydrogen, $C_1$-$C_3$alkyl, $C_1$-$C_3$cyanoalkyl or $C_1$-$C_3$alkoxy,

$R^{15}$ is $C_3$-$C_6$alkinyl, $C_2$-$C_6$alkenyl, $C_1$-$C_6$alkyl, $C_1$-$C_4$halogenoalkyl, $C_2$-$C_4$halogenoalkenyl or $C_1$-$C_4$alkyl, substituted by cyano, methoxy, ethoxy, nitro, $C_1$-$C_4$alkoxycarbonyl, methylthio, ethylthio, methylsulfonyl or ethylsulfonyl; or $C_3$-$C_4$alkenyl substituted by nitro, cyano, methoxy or ethoxy,

$R^{16}$ is hydrogen, $C_1$-$C_3$alkyl or $C_3$-$C_4$alkenyl,

$R^{17}$ is hydrogen, $C_1$-$C_3$alkyl, $C_1$-$C_3$cyanoalkyl or $C_1$-$C_3$alkoxy,

$R^{18}$ is $C_1$-$C_3$alkyl and $C_1$-$C_3$halogenoalkyl,

W is oxygen or sulfur,

Z is oxygen, sulfur, -SO- or $-SO_2-$ and

m is the number zero or one, or a salt of one of these compounds.

2. A compound according to claim 1, in which X is oxygen.

3. A compound according to claim 1, in which A is a group of the formula

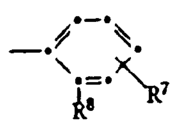

4. A compound according to claim 3, in which $R^7$ is hydrogen and $R^8$ is methoxycarbonyl, ethoxycarbonyl, dimethylaminosulfonyl, propoxy, ethoxy, difluoromethoxy, trifluoromethyl, chloroethoxy, methoxyethoxy, 2,2,2-trifluoroethoxy, 1,2-dichlorovinyloxy, nitro, fluorine, chlorine, bromine, methyl, methylthio, difluoromethylthio, chloroethylthio, $-O-SO_2CH_3$, allyloxy or methoxy.

5. A compound according to claim 1, in which $R^3$ is hydrogen.

6. A compound according to claim 1, in which $R^1$ is methyl or ethyl, $R^2$ is hydrogen, methoxy or methyl and E is the group $=CR^4-$.

7. A compound according to claim 1, in which $R^1$ is methyl or ethyl, $R^2$ is methoxy, ethoxy, methylthio, dimethylamino, methyl, trifluoromethyl, 2,2,2-trifluoroethoxy or ethyl and E is nitrogen.

8. A compound according to claim 6, in which $R^4$ is chlorine, bromine, methoxy, ethoxy, methyl, ethyl, methylthio, dimethylamino, trifluoromethyl or 2,2,2-trifluoroethoxy.

9. A compound according to claim 1, in which $R^1$ is methyl or ethyl, $R^2$ is hydrogen, methoxy or methyl, $R^3$ is hydrogen, X is oxygen, A is the group

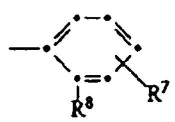

$R^7$ is hydrogen, $R^8$ is methoxycarbonyl, ethoxycarbonyl, dimethylaminosulfonyl, methoxy, ethoxy, difluoromethoxy, trifluoromethyl, chloroethoxy, methoxyethoxy, 2,2,2-trifluoroethoxy, 1,2-dichlorovinyloxy, nitro, fluorine, chlorine, bromine, methyl, methylthio, difluoromethylthio, chloroethylthio, $-O-SO_2CH_3$ or allyloxy, E is the group $=CR^4-$ and $R^4$ is chlorine, bromine, methoxy, ethoxy, methyl, ethyl, methylthio, dimethylamino, trifluoromethyl or 2,2,2-trifluoroethoxy.

10. A compound according to claim 1, in which $R^1$ is methyl or ethyl, $R^2$ is methoxy, ethoxy, methylthio, dimethylamino, methyl, trifluoromethyl, 2,2,2-trifluoroethoxy or ethyl, $R^3$ is hydrogen, E is nitrogen, X is oxygen, A is the group

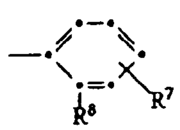
,

$R^7$ is hydrogen and $R^8$ is methoxycarbonyl, ethoxycarbonyl, dimethylaminosulfonyl, methoxy, ethoxy, difluoromethoxy, trifluoromethyl, chloroethoxy, methoxyethoxy, 2,2,2-trifluoroethoxy, 1,2-dichlorovinyloxy, nitro, $-O-SO_2CH_3$, fluorine, chlorine, bromine, methyl, methylthio, difluoromethylthio, chloroethylthio or allyloxy.

11. 6-[3-(2-Methoxycarbonylphenylsulfonyl)-ureido]-3-methoxy-4-methyl-1,2,4-triazin-5-one according to claim 1.

12. 6-[3-(2-Methoxycarbonylphenylsulfonyl)-ureido]-5-chloro-1-methylpyrazin-2-one according to claim 1.

13. A process for the preparation of a compound of the formula I, which comprises reacting an aminopyrazinone or aminotriazinone of the formula II

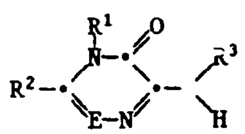
(II)

in which $R^1$, $R^2$, $R^3$ and E are as defined under formula I, with a sulfonyl isocyanate of the formula III

$$X = C = N\text{-}SO_2\text{-}A \quad (III)$$

in which A and X are as defined under formula I, and if appropriate converting the product into a salt.

14. A process for the preparation of a compound of the formula I, which comprises reacting an aminopyrazinone or aminotriazinone of the formula II according to claim 13 with a sulfonyl carbamate of the formula IV

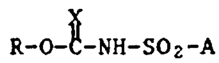 (IV)

in which A and X are as defined under formula I and R is phenyl, alkyl or substituted phenyl, in the presence of a base, and if appropriate converting the product into a salt.

15. A process for the preparation of a compound of the formula I, which comprises reacting a carbamate of the formula V

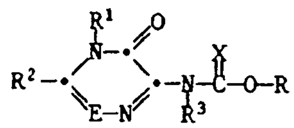 (V)

in which $R^1$, $R^2$, $R^3$, E and X are as defined under formula I and R is phenyl, alkyl or substituted phenyl, with a sulfonamide of the formula VI

$H_2N\text{-}SO_2\text{-}A$ (VI)

in which A is as defined under formula I, in the presence of a base, and if appropriate converting the product into a salt.

16. A process for the preparation of a salt of the formula I according to any one of claims 12 to 15, wherein a sulfonylurea of the formula I is reacted with an amine, an alkali metal or alkaline earth metal hydroxide or a quaternary ammonium base.

17. A herbicidal and plant growth-inhibiting composition, which contains, in addition to carriers and/or other additives, at least one substituted sulfonylurea of the formula I, claim 1, as the active substance.

18. A method of controlling undesirable plant growth, which comprises applying an effective amount of an active substance of the formula I according to claim 1 or of a composition containing this active substance to the plants or their environment.

19. A method of inhibiting plant growth, which comprises applying an effective amount of an active substance of the formula I according to claim 1 or of a composition containing this active substance to the plants or their environment.

20. A method of influencing plant growth for the purpose of increasing yield, which comprises applying an effective amount of an active substance of the formula I according to claim 1 or of a composition containing this active substance to the plants or their environment.

21. A method according to claim 18 for selective pre- or post-emergent control of weeds in crops of useful plants.

22. A method according to claim 19 for suppressing plant growth beyond the 2-leaf stage, wherein the active substance is used by the preemergence method.

23. A compound of the formula II

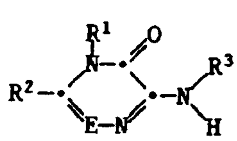 (II)

in which

E is nitrogen or $=CR^4$-,

$R^1$ is $C_1$-$C_4$alkyl,

$R^2$ is hydrogen, $C_1$-$C_3$alkyl, $C_1$-$C_3$halogenoalkyl, $C_1$-$C_3$alkoxy, $C_1$-$C_3$halogenoalkoxy, $C_1$-$C_3$alkylthio, $C_1$-$C_3$alkylsulfinyl, $C_1$-$C_2$alkoxyethoxy, $C_1$-$C_3$alkylsulfonyl, halogen or $-NR^5R^6$,

$R^3$ is hydrogen or $C_1$-$C_3$alkyl,

$R^4$ is hydrogen, $C_1$-$C_3$alkyl, $C_1$-$C_3$halogenoalkyl, $C_1$-$C_3$alkoxy, cyclopropyl, $C_1$-$C_3$halogenoalkoxy, $C_1$-$C_3$alkylthio, $C_2$-$C_4$alkoxyalkyl, $C_3$-$C_5$dialkoxymethyl, halogen or $-NR^5R^6$ and

$R^5$ and $R^6$ independently of one another are hydrogen or $C_1$-$C_3$alkyl, with the proviso that $R^3$ is $C_1$-$C_3$alkyl

if

a) at the same time E is nitrogen, $R^1$ is methyl and $R^2$ is methyl or methylthio; or
b) at the same time $R^1$ is methyl, $R^2$ is hydrogen and E is the group =CCl- or =CH-.

## Revendications

1. Aminopyrazinones et aminotriazinones de formule I

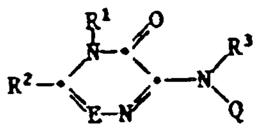

(I),

dans laquelle
E représente l'azote ou $=CR^4-$,
$R^1$ représente un groupe alkyle en C 1-C 4,
$R^2$ représente l'hydrogène, un groupe alkyle en C 1-C 3, halogénoalkyle en C 1-C 3, alcoxy en C 1-C 3, halogénoalcoxy en C 1-C 3, alkylthio en C 1-C 3, alkylsulfinyle en C 1-C 3, (alcoxy en C 1-C 2)-éthoxy, alkylsulfonyle en C 1-C 3, un halogène ou $-NR^5R^6$,
$R^3$ représente l'hydrogène ou un groupe alkyle en C 1-C 3,
Q représente un groupe

$$-\underset{\underset{X}{\|}}{C}-NH-SO_2-A,$$

$R^4$ représente l'hydrogène, un groupe alkyle en C 1-C 3, halogénoalkyle en C 1-C 3, alcoxy en C 1-C 3, cyclopropyle, halogénoalcoxy en C 1-C 3, alkylthio en C 1-C 3, alcoxyalkyle en C 2-C 4, dialcoxyméthyle en C 3-C 5, un halogène ou $-NR^5R^6$,
$R^5$ et $R^6$ représentent chacun, indépendamment l'un de l'autre, l'hydrogène ou un groupe alkyle en C 1-C 3,
X représente l'oxygène ou le soufre, et
A représente un groupe

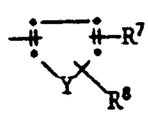

dans lequel
Y représente l'oxygène, le soufre, -CH=CH-, $-NR^9-$ ou $-CR^{10}=N-$,
$R^7$ représente l'hydrogène, un halogène, un groupe alkyle en C 1-C 3, alcoxy en C 1-C 3, nitro ou trifluorométhyle,
$R^8$ représente l'hydrogène, un halogène, un groupe alkyle en C 1-C 3, alcoxy en C 1-C 3, nitro, -C≡CH, ou l'un

des groupes $-\underset{\underset{W}{\|}}{C}-R^{11}$ , $-\underset{\underset{R^{11}}{|}}{C}$(alcoxy en C 1-C 3)$_2$,

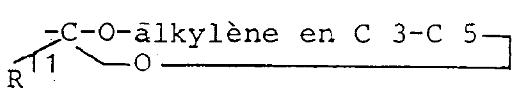

, $-\underset{\underset{O}{\|}}{C}-R^{12}$, $-SO_2-NR^{13}R^{14}$,

$-(Z)_m-R^{15}$ et $-O-SO_2R^{18}$,
$R^9$ et $R^{10}$ représentent chacun, indépendamment l'un de l'autre, l'hydrogène, un groupe alkyle en C 1-C 3 ou alcényle en C 2-C 4,

$R^{11}$ représente l'hydrogène, un groupe alkyle en C 1-C 3, halogénoalkyle en C 1-C 3, cycloalkyle en C 3-C 6 ou alcoxyalkyle en C 2-C 4,

$R^{12}$ représente un groupe alcoxy en C 1-C 6, alcényloxy en C 3-C 6, alcynyloxy en C 3-C 6, halogénoalcoxy en C 2-C 6, cyano-(alcoxy en C 1-C 4), alkylthio en C 1-C 6, alcénylthio en C 3-C 6, alcynylthio en C 3-C 6, cycloalcoxy en C 5-C 6, alcoxyalcoxy en C 2-C 6 ou $NR^{16}R^{17}$,

$R^{13}$ représente l'hydrogène, un groupe alkyle en C 1-C 3 ou alcényle en C 3-C 4,

$R^{14}$ représente l'hydrogène, un groupe alkyle en C 1-C 3, cyano-(alkyle en C 1-C 3) ou alcoxy en C 1-C 3,

$R^{15}$ représente un groupe alcynyle en C 3-C 6, alcényle en C 2-C 6, alkyle en C 1-C 6, halogénoalkyle en C 1-C 4, hologénoalcényle en C 2-C 4, alkyle en C 1-C 4 substitué par des groupes cyano, méthoxy, éthoxy, nitro, (alcoxy en C 1-C 4)-carbonyle, méthylthio, éthylthio, méthylsulfonyle ou éthylsulfonyle; ou alcényle en C 3-C 4 substitué par des groupes nitro, cyano, méthoxy ou éthoxy,

$R^{16}$ représente l'hydrogène, un groupe alkyle en C 1-C 3 ou alcényle en C 3-C 4,

$R^{17}$ représente l'hydrogène, un groupe alkyle en C 1-C 3, cyano-(alkyle en C 1-C 3) ou alcoxy en C 1-C 3,

$R^{18}$ représente un groupe alkyle en C 1-C 3 ou halogénoalkyle en C 1-C 3,

W représente l'oxygène ou le soufre,

Z représente l'oxygène, le soufre, -SO- ou $-SO_2-$, et

m est égal à 0 ou 1, et les sels de ces composés.

2. Composés selon la revendication 1, caractérisés en ce que X représente l'oxygène.

3. Composés selon la revendication 1, caractérisés en ce que A représente un groupe

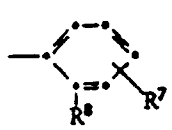

4. Composés selon la revendication 3, caractérisés en ce que $R^7$ représente l'hydrogène et $R^8$ un groupe méthoxycarbonyle, éthoxycarbonyle, diméthylaminosulfonyle, propoxy, éthoxy, difluorométhoxy, trifluorométhyle, chloréthoxy, méthoxyéthoxy, 2,2,2-trifluoréthoxy, 1,2-dichlorovinyloxy, nitro, le fluor, le chlore, le brome, un groupe méthyle, méthylthio, difluorométhylthio, chloréthylthio, $-O-SO_2CH_3$, allyloxy ou méthoxy.

5. Composés selon la revendication 1, caractérisés en ce que $R^3$ représente l'hydrogène.

6. Composés selon la revendication 1, caractérisés en ce que $R^1$ représente un groupe méthyle ou éthyle, $R^2$ l'hydrogène, un groupe méthoxy ou méthyle et E le groupe $=CR^4-$.

7. Composés selon la revendication 1, caractérisés en ce que $R^1$ représente un groupe méthyle ou éthyle, $R^2$ un groupe méthoxy, éthoxy, méthylthio, diméthylamino, méthyle, trifluorométhyle, 2,2,2-trifluoréthoxy ou éthyle, et E représente l'azote.

8. Composés selon la revendication 6, caractérisés en ce que $R^4$ représente le chlore, le brome, un groupe méthoxy, éthoxy, méthyle, éthyle, méthylthio, diméthylamino, trifluorométhyle ou 2,2,2-trifluoréthoxy.

9. Composés selon la revendication 1, caractérisés en ce que $R^1$ représente un groupe méthyle ou éthyle, $R^2$ l'hydrogène, un groupe méthoxy ou méthyle, $R^3$ l'hydrogène, X l'oxygène, A le groupe

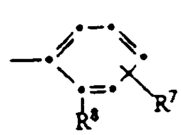

$R^7$ l'hydrogène , $R^8$ un groupe méthoxycarbonyle, éthoxycarbonyle, diméthylaminosulfonyle, méthoxy, éthoxy, difluorométhoxy, trifluorométhyle, chloréthoxy, méthoxyéthoxy, 2,2,2-trifluorothéxy, 1,2-dichlorovinyloxy, nitro, le fluor, le chlore, le brome, un groupe méthyle, méthylthio, difluorométhylthio, chloréthylthio, $-O-SO_2CH_3$ ou allyloxy, E le groupe $=CR^4-$ et $R^4$ le chlore, le brome, un groupe méthoxy, éthoxy, méthyle, éthyle, méthylthio, diméthylamino, trifluorométhyle ou 2,2,2-trifluoréthoxy.

10. Composés selon la revendication 1, caractérisés en ce que $R^1$ représente un groupe méthyle ou éthyle, $R^2$ un groupe méthoxy, éthoxy, méthylthio, diméthylamino, méthyle, trifluorométhyle, 2,2,2-trifluoréthoxy ou éthyle, $R^3$ représente l'hydrogène, E l'azote, X l'oxygène, A le groupe

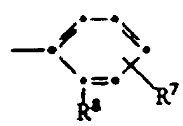

,

$R^7$ l'hydrogène et $R^8$ un groupe méthoxycarbonyle, éthoxycarbonyle, diméthylaminosulfonyle, méthoxy, éthoxy, difluorométhoxy, trifluorométhyle, chloréthoxy, méthoxyéthoxy, 2,2,2-trifluoréthoxy, 1,2-dichlorovinyloxy, nitro, $-O-SO_2CH_3$, le fluor, le chlore, le brome, un groupe méthyle, méthylthio, difluorométhylthio, chloréthylthio ou allyloxy.

11. La 6-[3-(2-méthoxycarbonylphénylsulfonyl)-uréido]-3-méthoxy-4-méthyl-1,2,4-triazine-5-one selon revendication 1.

12. La 6-[3-(2-méthoxycarbonylphénylsulfonyl)-uréido]-5-chloro-1-méthylpyrazine-2-one selon revendication 1.

13. Procédé de préparation des composés de formule I, caractérisé en ce que l'on fait réagir une aminopyrazinone ou aminotriazinone de formule II

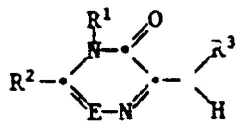

(II)

dans laquelle $R^1$, $R^2$, $R^3$ et E ont les significations indiquées en réference à la formule I, avec un sulfonylisocyanate de formule III

$$X = C = N\text{-}SO_2\text{-}A \quad (III)$$

dans laquelle A et X ont les significations indiquées en référence à la formule I, et le cas échéant on convertit en un sel.

14. Procédé de préparation des composés de formule I, caractérisé en ce que l'on fait réagir une aminopyrazinone ou aminotriazinone de formule II de la revendication 13, en présence d'une base, avec un sulfonylcarbamate de formule IV

$$R-O-\overset{\overset{\displaystyle X}{\|}}{C}-NH-SO_2-A \qquad (IV)$$

dans laquelle A et X ont les significations indiquées en référence à la formule I et R représente un groupe phényle, alkyle ou phényle substitué, et le cas échéant on convertit en un sel.

15. Procédé de préparation des composés de formule I, caractérisé en ce que l'on fait réagir un carbamate de formule V

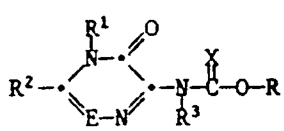

(V)

dans laquelle $R^1$, $R^2$, $R^3$, E et X ont les significations indiquées en référence à la formule I et R représente un groupe phényle, alkyle ou phényle substitué, en présence d'une base, avec un sulfonamide de formule VI

$$H_2N\text{-}SO_2\text{-}A \quad (VI)$$

dans laquelle A a les significations indiquées en référence à la formule I, et le cas échéant on convertit en un sel.

16. Procédé de préparation des sels de formule I selon l'une des revendications 12 à 15, caractérisé en ce que l'on fait réagir une sulfonylurée de formule I avec une amine, un hydroxyde de métal alcalin ou alcalino-terreux ou une base d'ammonium quaternaire.

17. Un produit herbicide ou inhibiteur de la croissance des végétaux, caractérisé en ce qu'il contient, avec des véhicules et/ou d'autres additifs, au moins une substance active consistant en une sulfonylurée substituée

de formule I, revendication 1.

18. Procédé pour combattre les croissances de végétaux indésirables, caractérisé en ce que l'on applique une substance active de formule I selon revendication 1 ou un produit contenant cette substance active, en quantité efficace, sur les végétaux ou leur habitat.

19. Procédé pour imbiber la croissance des végétaux, caractérisé en ce que l'on applique une substance active de formule I selon revendication 1 ou un produit contenant cette substance active, en quantité efficace, sur les végétaux ou leur habitat.

20. Procédé pour agir sur la croissance des végétaux en vue d'augmenter les rendements, caractérisé en ce que l'on applique une substance active de formule I selon revendication 1 ou un produit contenant cette substance active, en quantité efficace, sur les végétaux ou leur habitat.

21. Procédé selon la revendication 18, pour la lutte sélective en pré-levée ou en post-levée contre les végétaux adventices dans les cultures de végétaux utiles.

22. Procédé selon la revendication 19, pour inhiber la croissance des végétaux au-delà du stade deux feuilles, caractérisé en ce que l'on applique les substances actives en pré-levée.

23. Composés de formule II

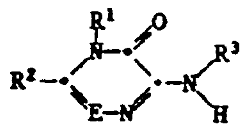

(II)

dans laquelle

E représente l'azote ou $=CR^4-$,

$R^1$ représente un groupe alkyle en C 1-C 4,

$R^2$ représente l'hydrogène, un groupe alkyle en C 1-C 3, halogénoalkyle en C 1-C 3, alcoxy en C 1-C 3, halogénoalcoxy en C 1-C 3, alkylthio en C 1-C 3, alkylsulfinyle en C 1-C 3, (alcoxy en C 1-C 2)-éthoxy, alkylsulfonyle en C 1-C 3, un halogène ou $-NR^5R^6$,

$R^3$ représente l'hydrogène ou un groupe alkyle en C 1-C 3,

$R^4$ représente l'hydrogène, un groupe alkyle en C 1-C 3, halogénoalkyle en C 1-C 3, alcoxy en C 1-C 3, cyclopropyle, halogénoalcoxy en C 1-C 3, alkylthio en C 1-C 3, alcoxyalkyle en C 2-C 4, dialcoxyméthyle en C 3-C 5, un halogène ou $-NR^5R^6$;

$R^5$ et $R^6$ représentent chacun, indépendamment l'un de l'autre, l'hydrogène ou un groupe alkyle en C 1-C 3, sous réserve que $R^3$ représente un groupe alkyle en C 1-C 3 lorsque :

a) simultanément E represente l'azote, $R^1$ un groupe méthyle et $R^2$ un groupe méthyle ou méthylthio; ou bien

b) simultanément $R^1$ représente un groupe méthyle, $R^2$ l'hydrogène et E le groupe =CCl- ou =CH-.